(19) Europäisches Patentamt
European Patent Office
Office européen des brevets

(11) EP 0 753 506 B1

(12) EUROPÄISCHE PATENTSCHRIFT

(45) Veröffentlichungstag und Bekanntmachung des Hinweises auf die Patenterteilung:
**18.08.1999 Patentblatt 1999/33**

(51) Int Cl.[6]: **C07C 215/46**, A61K 31/135, C07C 217/54, C07C 219/28

(21) Anmeldenummer: **96110104.5**

(22) Anmeldetag: **22.06.1996**

(54) **6-Dimethylaminomethyl-1-phenyl-cyclo-hexanverbindungen als pharmazeutische Wirkstoffe**

6-Dimethylaminomethyl-1-phenyl-cyclo-hexane compounds as pharmaceutical agents

Composés 6-diméthylaminométhyl-1-phényl-cyclohexane comme agents pharmaceutiques

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **11.07.1995 DE 19525137**

(43) Veröffentlichungstag der Anmeldung:
**15.01.1997 Patentblatt 1997/03**

(73) Patentinhaber: **Grünenthal GmbH**
**52078 Aachen (DE)**

(72) Erfinder:
- **Buschmann, Helmut, Dr.**
  **52066 Aachen (DE)**
- **Strassburger, Wolfgang, Prof.**
  **52146 Würselen (DE)**
- **Selve, Norma, Dr.**
  **52078 Aachen (DE)**
- **Friderichs, Elmar, Dr.**
  **52223 Stolberg (DE)**

(56) Entgegenhaltungen:
**GB-A- 997 399** **US-A- 3 652 589**

- **CHEMICAL AND PHARMACEUTICAL BULLETIN, Bd. 32, Nr. 6, 1984, TOKYO JP, Seiten 2279-2289, XP002016125 TAKESHI KATO ET AL.: "Synthesis and analgesic activity of cyclohexenylmethyl- amines and related compounds"**
- **CHEMICAL ABSTRACTS, vol. 88, no. 21, 22.Mai 1978 Columbus, Ohio, US; abstract no. 145970t, FLICK, K ET AL.: "Studies on chemical structure and analgetic activity of phenyl substituted aminomethylcyclohexanols" Seite 22; Spalte 2; XP002016126 & ARZNEIM. FORSCH., Bd. 28, Nr. 1A, 1978, Seiten 107-113,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 753 506 B1

Printed by Jouve, 75001 PARIS (FR)

## Beschreibung

Die Erfindung betrifft 6-Dimethylaminomethyl-1-phenyl-cyclohexanverbindungen, Verfahren zu deren Herstellung sowie die Verwendung dieser Verbindungen in Arzneimitteln.

Die Behandlung chronischer und nicht chronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für die Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Opioide werden seit vielen Jahren zur Schmerzbehandlung eingesetzt, obwohl sie eine Reihe von Nebenwirkungen, beispielsweise Abhängigkeit, Atemdepression, gastrointestinale Hemmwirkung und Obstipation, hervorrufen. Sie können daher nur unter besonderen Vorsichtsmaßnahmen, beispielsweise speziellen Verordnungsvorschriften über einen längeren Zeitraum oder in höheren Dosierungen gegeben werden (Goodman, Gilman "The Pharmacological Basis of Therapeutics" Pergamon Press, New York, 1990).

Tramadolhydrochlorid - (1RS,2RS)-2[(Dimethylamino)methyl]-1-(3-methoxyphenyl)-cyclohexanol, Hydrochlorid - nimmt unter den zentral wirksamen Analgetika eine Sonderstellung ein, da dieser Wirkstoff eine starke Schmerzhemmung ohne die für Opioide bekannten Nebenwirkungen hervorruft (J. Pharmacol. Exp. Ther. 267, 331 (1993)). Tramadol ist ein Racemat und besteht aus gleichen Mengen an (+)- und (-)-Enantiomer. In vivo bildet der Wirkstoff den Metaboliten O-Desmethyl-tramadol, der ebenfalls als Enantiomerengemisch vorliegt. Untersuchungen haben ergeben, daß sowohl die Enantiomeren von Tramadol als auch die Enantiomeren der Tramadolmetabolite an der analgetischen Wirkung beteiligt sind (J. Pharmacol. Exp. Ther. 260, 275 (1992)).

Aus Chem. Pharm. Bull. 32, 2279 (1984) sind Verbindungen der Formel

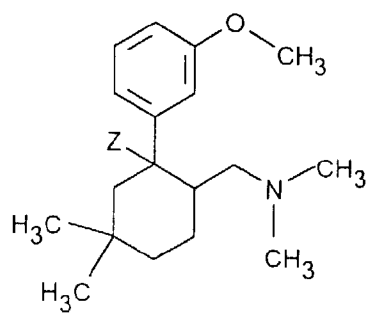

bekannt, in der Z H oder OH bedeutet. Diese Substanzen besitzen eine analgetische Wirkung, die im Vergleich zu Tramadol deutlich schwächer ist.

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen. Darüber hinaus sollten die zu entwickelnden Substanzen nicht die während der Behandlung mit Tramadol in manchen Fällen auftretenden Nebenwirkungen, beispielsweise Übelkeit und Erbrechen, besitzen.

Es wurde nun gefunden, daß die an die zu entwickelnden Substanzen gestellten Anforderungen von bestimmten 6-Dimethylaminomethyl-1-phenyl-cyclohexanverbindungen erfüllt werden. Diese Verbindungen zeichnen sich durch eine ausgeprägte analgetische Wirkung aus, die im Vergleich zu Tramadol und zu den aus Arzneim.-Forsch./ Drug Res. 28 (IA) 107 (1978) bekannten Verbindungen der Formel

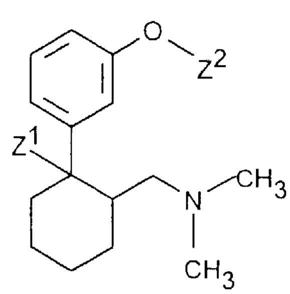

mit $Z^1$ H, OH oder Cl und $Z^2$ $CH_3$ oder $Z^1$ OH und $Z^2$ H deutlich verstärkt ist.

Gegenstand der Erfindung sind dementsprechend 6-Dimethylaminomethyl-1-phenyl-cyclohexanverbindungen der Formel I

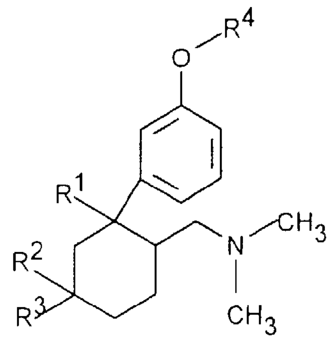

in der

$R^1$ H, OH, Cl oder F ist,

$R^2$ und $R^3$ gleich oder verschieden sind und H, $C_{1-4}$-Alkyl, Benzyl, $CF_3$, OH, $OCH_2$-$C_6H_5$, O-$C_{1-4}$-Alkyl, Cl oder F bedeuten mit der Maßgabe, daß wenigstens einer der Reste $R^2$ oder $R^3$ H bedeutet,

$R^4$ H, $CH_3$, PO($OC_{1-4}$-Alkyl)$_2$, CO($OC_{1-5}$-Alkyl), CO-NH-$C_6H_4$-$C_{1-3}$-Alkyl, CO-$C_6H_4$-$R^5$, CO-$C_{1-5}$-Alkyl, CO-$CHR^6$-$NHR^7$ oder eine unsubstituierte oder substituierte Pyridyl-, Thienyl-, Thiazoyl- oder Phenylgruppe bedeutet,

$R^5$ OC(O)$C_{1-3}$-Alkyl in ortho-Stellung oder $CH_2$-N($R^8$)$_2$ in meta- oder para-Stellung, wobei $R^8$ $C_{1-4}$-Alkyl oder beide Reste $R^8$ zusammen mit N den 4-Morpholino-Rest darstellen, bedeutet, und

$R^6$ und $R^7$ gleich oder verschieden sind und H oder $C_{1-6}$-Alkyl bedeuten,

mit der Maßgabe, daß wenn beide Reste $R^2$ und $R^3$ H bedeuten, $R^4$ nicht $CH_3$ ist, wenn $R^1$ H, OH oder Cl bedeutet oder $R^4$ nicht H ist, wenn $R^1$ OH bedeutet,
in Form ihrer Basen oder Salze von physiologisch verträglichen Säuren.

Bevorzugte 6-Dimethylaminomethy1-1-phenyl-cyclohexanverbindungen entsprechen der Formel I mit $R^1$ H, OH oder F. Besonders bevorzugt werden 6-Dimethylaminomethyl-1-phenyl-cyclohexanverbindungen in Form ihrer Diastereomeren mit der Konfiguration der Formel Ia, in der der Phenylring und die Dimethylaminomethyl-Gruppe trans zueinander stehen:

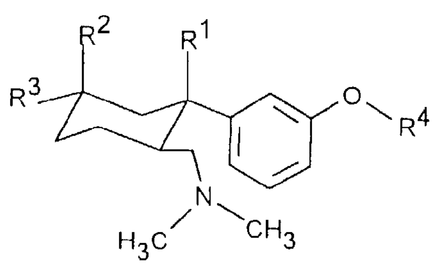

Weiterer Erfindungsgegenstand ist ein Verfahren zur Herstellung von 6-Dimethylaminomethyl-1-phenyl-cyclohexanverbindungen der Formel I, in der $R^1$ OH bedeutet und $R^2$ und $R^3$ gleich oder verschieden sind und H, $C_{1-4}$-Alkyl, Benzyl, $CF_3$, Cl oder F bedeuten mit der Maßgabe, daß mindestens einer der Reste $R^2$ oder $R^3$ H ist und $R^4$ H, $CH_3$ oder eine unsubstituierte oder substituierte Pyridyl-, Thienyl-, Thiazoyl- oder Phenylgruppe bedeutet, mit der Maßgabe, daß $R^4$ weder $CH_3$ noch H ist, wenn beide Reste $R^2$ und $R^3$ H bedeuten, wobei das Verfahren dadurch gekennzeichnet ist, daß ein β-Dimethylaminoketon der Formel II

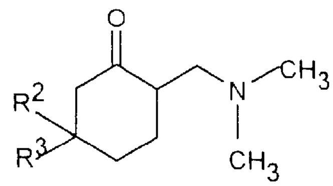

mit einer metallorganischen Verbindung der Formel III

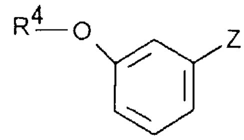

in der Z MgCl, MgBr, MgI oder Li bedeutet, zu einer Verbindung der Formel I umgesetzt wird.

Die Reaktion eines β-Dimethylaminoketons mit einer Grignard-Verbindung der Formel III oder mit einer lithiumorganischen Verbindung der Formel III kann in einem aliphatischen Ether, beispielsweise Diethylether und/oder Tetrahydrofuran, bei einer Temperatur zwischen -70° C und +60° C durchgeführt werden. Lithiumorganische Verbindungen der Formel III lassen sich durch Umsetzung einer Verbindung der Formel III, in der Z Cl Br oder I bedeutet, mit beispielsweise einer n-Butyllithium/Hexan-Lösung durch Halogen/Lithiumaustausch erhalten. Bei der Umsetzung eines β-Dimethylaminoketons der Formel II mit einer metallorganischen Verbindung werden 6-Dimethylaminomethyl-1-phenyl-cyclohexanverbindungen mit der bevorzugten relativen Konfiguration der Formel Ia erhalten.

β-Dimethylaminoketone der Formel II sind aus Ketonen der Formel IV

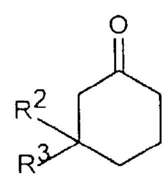

durch Umsetzung mit Dimethylaminohydrochlorid und Formaldehyd in Eisessig oder in einem $C_{1-4}$-Alkylalkohol oder durch Umsetzung mit Dimethylammoniummethylenchlorid in Acetonitril unter Acetylchloridkatalyse erhältlich (Synthesis 1973, 703; Tietze, Eicher in "Reaktionen und Synthesen im Organisch-Chemischen Praktikum", Thieme-Verlag, Stuttgart 1991, Seite 189). Die bei der Aminomethylierungsreaktion entstehendenden diastereomeren β-Dimethylaminoketone können entweder durch säulenchromatographische Trennung oder durch fraktionierte Kristallisation ihrer Hydrochloride aus einem organischen Lösungsmittel, beispielsweise 2-Butanon und/oder Aceton, diastereomerenrein erhalten werden.

Ferner ist Erfindungsgegenstand ein Verfahren zur Herstellung von 6-Dimethylaminomethyl-1-phenyl-cyclohexanverbindungen der Formel I, in der $R^1$ OH ist, einer der Reste $R^2$ oder $R^3$ H und der andere OH, O-$C_{1-4}$-Alkyl oder $OCH_2C_6H_5$ bedeuten und $R^4$ H, $CH_3$ oder eine unsubstituierte oder substituierte Pyridyl-, Thienyl-, Thiazoyl- oder Phenylgruppe bedeutet, welches dadurch gekennzeichnet ist, daß man ein β-Dimethylaminoketon mit spirocyclischer Acetalstruktur der Formel V

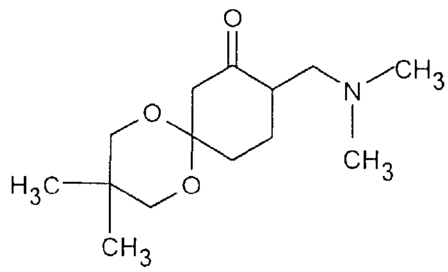

mit einer metallorganischen Verbindung der Formel III

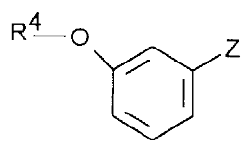

in der Z MgCl, MgBr, MgI oder Li bedeutet, zu einer Verbindung der Formel VI

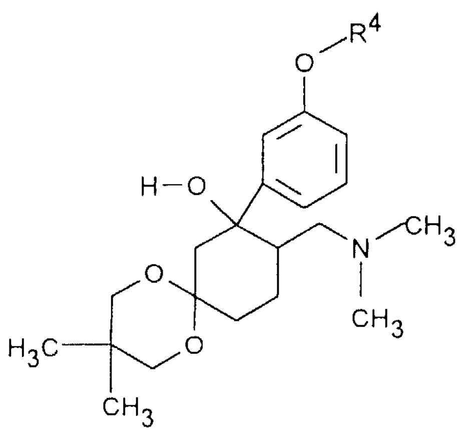

umsetzt, die erhaltene Verbindung der Formel VI durch protonen-katalysierte Deacetalisierung in das entsprechende Ketonderivat der Formel VIII

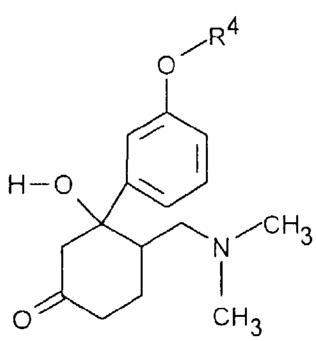

überführt und das erhaltene Ketonderivat anschließend mit einem komplexen Alkalimetallhydrid zu einer Verbindung der Formel I, in der einer der Reste $R^2$ oder $R^3$ OH bedeutet, reduziert und gewünschtenfalls die durch Reduktion erhaltene Verbindung der Formel I nach Überführung in ein Alkalisalz mit einem $C_{1-4}$-Alkyl- oder Benzylhalogenid in eine Verbindung der Formel I, in der einer der Reste $R^2$ oder $R^3$ O-$C_{1-4}$-Alkyl oder $OCH_2C_6H_5$ bedeutet, überführt.

Die Reduktion einer Verbindung der Formel VIII wird vorzugsweise mit Natriumborhydrid oder Lithiumaluminiumhydrid in einem organischen Lösungsmittel, beispielsweise Tetrahydrofuran, Diethylether und/oder einem $C_{2-4}$-Alkylalkohol durchgeführt. Sofern nach dem erfindungsgemäßen Verfahren eine Verbindung mit $R^2$ oder $R^3$ $OC_{1-4}$-Alkyl oder $OCH_2Ph$ erhalten werden soll, wird die durch Reduktion erhaltene Verbindung mit einem Alkalihydrid, beispielsweise Natrium- und/oder Kaliumhydrid, in einem Lösungsmittel, wie Dimethylformamid in die entsprechende Alkalisalzverbindung überführt und anschließend mit einem $C_{1-4}$-Alkyl- oder Benzylhalogenid umgesetzt.

β-Dimethylaminoketone mit spirocyclischer Acetalstruktur der Formel V sind aus 9-Dimethylaminomethyl-3.3-dimethyl-1.5-dioxa-spiro[5.5]undecan-8-on der Formel VII

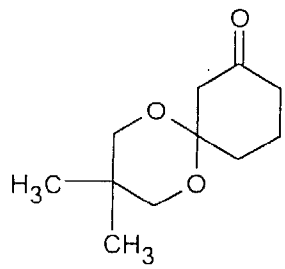

welches durch gezielte Monoacetalisierung von Cyclohexan-1,3-dion zugänglich ist, durch Umsetzung mit Dimethylammoniumethylenchlorid in Acetonitril unter Acetylchlorid-Katalyse erhältlich (Synthesis 1973, 703; Tietze, Eicher in "Reaktionen und Synthesen im Organisch-Chemischen Praktikum", Thieme-Verlag, Stuttgart 1991, Seite 189).

Weiterer Erfindungsgegenstand ist ein Verfahren zur Herstellung von 6-Dimethylaminomethyl-1-phenyl-cyclohexanverbindungen der Formel I, in der $R^1$ H ist und $R^2$ und $R^3$ gleich oder verschieden sind und H, $C_{1-4}$-Alkyl, Benzyl, $CF_3$, $OCH_2C_6H_5$ oder F bedeuten mit der Maßgabe, daß wenigstens einer der Reste $R^2$ oder $R^3$ H ist, und $R_4$ H, $CH_3$ oder eine unsubstituierte oder substituierte Pyridyl-, Thienyl-Thiazoyl- oder Phenylgruppe bedeutet, welches dadurch gekennzeichnet ist, daß eine Verbindung der Formel I, in der $R^1$ Cl ist, mit Zinkborhydrid, Zinkcyanoborhydrid oder Zinncyanoborhydrid in einem Ether oder eine Verbindung der Formel I, in der $R^1$ OH ist, mit Raney-Nickel in einem $C_{2-4}$-Alkylalkohol umgesetzt wird.

Die Reaktion einer Verbindung der Formel I, in der $R^1$ Cl ist, mit einem Borhydrid wird vorzugsweise in Diethylether und/oder Tetrahydrofuran bei einer Temperatur zwischen 0 und 30° C durchgeführt. Die Umsetzung einer Verbindung der Formel I, in der $R^1$ OH ist, mit Raney-Nickel wird vorzugsweise in einem $C_{2-4}$-Alkylalkohol bei einer Temperatur zwischen 70 und 100° C durchgeführt (J. Org. Chem. 59, 6895 (1994) und Angew. Chem. 95, 568 (1983)).

Cyclohexanverbindungen der Formel I, in der $R^1$ H ist, einer der Reste $R^2$ oder $R^3$ H und der andere Cl bedeutet und $R^4$ H, $CH_3$ oder eine unsubstituierte oder substituierte Pyridyl-, Thienyl-, Thiazoyl- oder Phenylgruppe darstellt, sind aus den entsprechenden Cyclohexanverbindungen der Formel I, in der einer der Reste $R^2$ oder $R^3$ H und der andere OH ist und $R^1$ und $R^4$ eine der vorgenannten Bedeutungen haben, durch Umsetzung mit Thionylchlorid oder Salzsäure/Zinkchlorid in an sich bekannter Weise erhältlich (J. Chem. Src. 1943, 636; J. Org. Chem. 17, 1116 (1952)).

Erfindungsgegenstand ist ferner ein Verfahren zur Herstellung von 6-Dimethylaminomethyl-1-phenyl-cyclohexanverbindungen der Formel I, in der $R^1$ H, $R^2$ und $R^3$ gleich oder verschieden sind und H, $C_{1-4}$-Alkyl, Benzyl, $CF_3$ oder F bedeuten, mit der Maßgabe, daß wenigstens einer der Reste $R^2$ oder $R^3$ H ist, und $R^4$ $CH_3$ bedeutet, welches dadurch gekennzeichnet ist, daß eine Verbindung der Formel I mit $R^1$ Cl in Gegenwart eines Palladiumkatalysators in einem $C_{1-4}$-Alkylalkohol hydriert wird. Die Hydrierung wird vorzugsweise bei einem Druck zwischen 1 und 100 bar und einer Temperatur zwischen 20 bis 80° C durchgeführt.

Verbindungen der Formel I, in der $R^1$ H ist, $R^2$ und $R^3$ gleich oder verschieden sind und H, $C_{1-4}$-Alkyl, Benzyl, $CF_3$ oder F bedeuten und $R^4$ H ist, lassen sich auch aus den entsprechenden Methoxyphenyl-Verbindungen durch mehrstündiges Erhitzen mit konzentrierter Bromwasserstoffsäure erhalten (Chem. Rev. 54, 615 (1954); J. Am. Chem. Soc. 74, 1316 (1952)).

Die Cyclohexanverbindungen der Formel I, in der $R^1$ Cl ist und keiner der Reste $R^2$ und $R^3$ OH bedeutet, sind durch Umsetzung einer Verbindung der Formel I, in der $R^1$ OH ist, in Form der freien Base oder als Hydrochlorid mit Thionylchlorid in Abwesenheit eines Lösungsmittels bei einer Temperatur zwischen 0 und 20° C erhältlich. Bei diesem Verfahren verläuft der Chloraustausch unter Erhalt der Konfiguration. Cyclohexanverbindungen der Formel I mit $R^1$ Cl und $R^2$ oder $R^3$ OH sind aus den entsprechenden Verbindungen mit $R^1$ Cl und $R^2$ oder $R^3$ $OCH_2C_6H_5$ in an sich bekannter Weise erhältlich.

Ferner ist Erfindungsgegenstand ein Verfahren zur Herstellung von 6-Dimethylaminomethyl-1-phenyl-cyclohexanverbindungen der Formel I, in der $R^1$ F ist, $R^2$ und $R^3$ gleich oder verschieden sind und H, $C_{1-4}$-Alkyl, Benzyl,

$CF_3$, $OCH_2$-$C_6H_5$, Cl oder F bedeuten mit der Maßgabe, daß wenigstens einer der Reste $R^2$ oder $R^3$ H ist und $R^4$ $CH_3$ oder eine unsubstituierte oder substituierte Pyridyl-, Thienyl-, Thiazoyl- oder Phenylgruppe bedeutet, welches dadurch gekennzeichnet ist, daß eine Verbindung der Formel I, in der $R^1$ OH ist, mit Dimethylamino-schwefeltrifluorid umgesetzt wird. Vorzugsweise wird die Umsetzung in einem organischen Lösungsmittel, beispielsweise Dichlormethan, 1,1,2-Trichlorethan und/oder Toluol bei einer Temperatur zwischen -50° C und +30° C durchgeführt (Org. Reac. 35, 513 (1988)).

Verbindungen der Formel I, in der $R^1$ F ist, $R^2$ und $R^3$ gleich oder verschieden sind und H, $C_{1-4}$-Alkyl, Benzyl, $CF_3$, $OCH_2$-$C_6H_5$, Cl oder F bedeuten, mit der Maßgabe, daß wenigstens einer der Reste $R^2$ und $R^3$ H ist und $R^4$ H bedeutet, lassen sich durch Umsetzung von Verbindungen der Formel I, in der $R^1$ OH und $R^4$ eine Trialkylsilylgruppe bedeutet, mit Dimethylaminoschwefeltrifluorid und anschließender Silyletherspaltung mit wäßrigen Mineralsäuren erhalten. Eine bevorzugte Trialkylsilylgruppe ist die Dimethyl-tert.-butylsilylgruppe.

Eine weitere Möglichkeit, 6-Dimethylaminomethyl-1-phenyl-cyclohexanverbindungen der Formel I zu erhalten, in der $R^1$ OH oder H, $R^4$ H und keiner der beiden Reste $R^2$ und $R^3$ Cl, F oder $CF_3$ bedeuten, besteht in der selektiven Etherspaltung mit Diisobutylaluminiumhydrid einer 6-Dimethylaminomethyl-1-(3-methoxyphenyl)-cyclohexanverbindung, die vorzugsweise in einem aromatischen Kohlenwasserstoff, beispielsweise Toluol, bei einer Temperatur zwischen 60 und 130° C durchgeführt wird (Synthesis 1975, 617).

Desweiteren lassen sich 6-Dimethylaminomethyl-1-phenyl-cyclohexanverbindungen der Formel I, in der $R^1$ OH, H oder F und $R^4$ H bedeuten und $R^2$ und $R^3$ gleich oder verschieden sind und H, $C_{1-4}$-Alkyl, Benzyl, $CF_3$, F, Cl, OH oder O-$C_{1-4}$-Alkyl bedeuten, aus den entsprechenden 6-Dimethylaminomethyl-1-(3-benzyloxy-phenyl)-cyclohexanverbindungen durch reduktive Debenzylierung erhalten. Die Debenzylierung wird vorzugsweise in Gegenwart von Platin oder Palladium auf einem Trägermaterial in Gegenwart von Wasserstoff in einem Lösungsmittel, beispielsweise Essigsäure und/oder einem $C_{1-4}$-Alkylalkohol, bei einem Druck zwischen 1 und 100 bar und einer Temperatur zwischen 20 und 100° C durchgeführt.

6-Dimethylaminomethyl-1-phenyl-cyclohexanverbindungen der Formel I, in der $OR^4$ eine Phosphat-, Carbonat-, Carbamat-, Carboxylat-, Aryloxy- oder Heteroaryloxygruppe bedeutet, lassen sich durch Umsetzung der entsprechenden 6-Dimethylaminomethyl-1-(3-hydroxyphenyl)-cyclohexanverbindungen in Form ihrer Alkalisalze mit einem Alkalisalz eines Dialkylchlorophosphates, mit einem Alkylchloroformiat, mit einem Aryl- oder Heteroarylisocyanat, mit einem Carbonsäurechlorid oder einem Aryl- oder Heteroarylhalogenid erhalten. Die Umsetzungen werden üblicherweise in einem Lösungsmittel, beispielsweise Toluol, Dichlormethan, Diethylether und/oder Tetrahydrofuran bei Temperaturen zwischen -15° C und +110° C durchgeführt (Drugs of the Future 16, 443 (1991); J. Med. Chem. 30, 2008 (1989) und 32, 2503 (1989); J. Org. Chem. 43, 4797 (1978); Tetrahedron Lett. 1977, 1571; J. Pharm. Sci. 57, 774 (1968)). Die Umsetzungen mit einem Aryl- oder Heteroarylhalogenid werden in Gegenwart von Kupferpulver und/oder einem Kupfer-I-halogenid als Katalysator durchgeführt.

6-Dimethylaminomethyl-1-phenyl-cyclohexanverbindungen der Formel I, in der $OR^4$ eine $\alpha$-Aminosäuregruppe ist, lassen sich durch Umsetzung der entsprechenden 6-Dimethylaminomethyl-1-(3-hydroxyphenyl)-cyclohexanverbindung mit der entsprechenden 2-t-Butoxycarbonylamino-carbonsäure unter Verwendung von Triethylamin und Kupplungsreagenzien, beispielsweise Benzotriazol-1-yl-oxy-tripyrrolidinophosphonium-hexafluorophosphat, in einem Lösungsmittel, beispielsweise Dichlormethan, erhalten.

Die erfindungsgemäßen Verbindungen lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in Gegenwart von Wasser in einem der vorgenannten Lösungsmittel.

Die erfindungsgemäßen Verbindungen haben eine ausgeprägte analgetische Wirkung und sind toxikologisch unbedenklich. Sie eignen sich daher als pharmazeutische Wirkstoffe. Dementsprechend ist Erfindungsgegenstand auch die Verwendung einer 6-Dimethylaminomethyl-1-phenyl-1-cyclohexanverbindung der Formel I als Wirkstoff in Arzneimitteln, vorzugsweise als Wirkstoff in Schmerzmitteln.

Erfindungsgemäße Arzneimittel enthalten neben mindestens einer 6-Dimethylaminomethyl-1-phenyl-cyclohexanverbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal und örtlich, zum Beispiel auf Infektionen an der Haut, an den Schleimhäuten und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot in gelöster Form oder in einem Pflaster, gegebenenfalls unter Zusatz von die Hauptpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 10 bis 500 mg pro kg wenigstens einer 6-Dimethylaminomethyl-1-phenyl-cyclohexanverbindung der Formel I appliziert.

## Beispiele

Soweit nicht anders angegeben, wurde Petrolether mit dem Siedebereich von 50 - 70° C benutzt. Die Angabe Ether bedeutet Diethylether.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Racemattrennungen wurden auf einer Chiracel OD Säule durchgeführt.

Die Mischungsverhältnisse der Laufmittel für alle chromatographischen Untersuchungen sind stets in Volumen/Volumen angegeben.

RT bedeutet Raumtemperatur, Schmp. Schmelzpunkt.

### Beispiel 1

(-)-(1S,2S)-3-(2-Dimethylaminomethyl-1-fluoro-cyclohexyl)-phenol, Hydrochlorid (**-1**)

1. Stufe:

(-)-(1S,2S)-1-(3-Benzyloxo-phenyl)-2-dimethylaminomethyl-cyclohexanol, Hydrochlorid (**-2**)

Aus (-)-(1S,2S)-3-(2-Dimethylaminomethyl-1-hydroxy-cyclohexyl)-phenol, Hydrochlorid wurde mit wäßriger Natriumhydrogencarbonatlösung/Dichlormethan die Base freigesetzt und nach Trocknung der Lösung Dichlormethan destillativ entfernt. 135 g (545 mmol) Base wurden in 675 ml trockenem Dimethylformamid gelöst und mit 29,1 g 50 %-igem Natriumhydrid in mehreren Portionen versetzt. Nach Zugabe von 69 ml (594 mmol) Benzoylchlorid wurde drei Stunden auf 70° C erhitzt. Anschließend wurde auf Raumtemperatur abgekühlt und das Reaktionsgemisch auf Eiswasser gegossen. Es wurde dreimal mit je 150 ml Ethylacetat extrahiert. Nach Trocknung der organischen Phasen über Magnesiumsulfat wurde das Lösungsmittel abdestilliert. Der Rückstand (204 g) wurde in 1000 ml 2-Butanon aufgenommen und mit 76 ml (600 mmol) Trimethylchlorsilan und 10,9 ml Wasser versetzt. Bei Raumtemperatur kristallisierten 190 g (93 % der Theorie) Hydrochlorid (**-2**) mit einem Schmelzpunkt von 207 - 210° C aus.

$[\alpha]_D^{RT}$ = -27,0° (c = 1,02; Methanol)

2. Stufe:

(-)-(1S,2S)-[2-(3-Benzyloxy-phenyl)-2-fluoro-cyclohexylmethyl]-dimethyl-amin (**-3**)

Zu einer Lösung von 80,6 g (500 mmol) Diethylamino-schwefeltrifluorid in 450 ml trockenem Dichlormethan wurden bei -40° C 147,7 g (435 mmol) (-**2**), gelöst in 1.500 ml trockenem Dichlormethan, getropft. Nach vollständiger Zugabe wurde 120 Minuten bei dieser Temperatur gerührt und anschließend auf Raumtemperatur erwärmt. Nach weiterem einstündigen Rühren bei Raumtemperatur wurde auf 0 - 5° C gekühlt und mit 500 ml Wasser hydrolysiert. Die wäßrige Phase wurde zweimal mit 200 ml Dichlormethan extrahiert. Nach Trocknung der organischen Phasen wurde das Lösungsmittel destillativ entfernt. Das erhaltene Rohgemisch (185 g) wurde in vier Portionen aufgeteilt. Jede Portion wurde auf eine 8 x 50 cm Säule, gefüllt mit Kieselgel, gegeben und mit Ethylacetat/Methanol = 1 : 1 eluiert. Insgesamt wurden 103 g (69 % der Theorie) Base (-**3**) als hellgelbes viskoses Öl erhalten.

3. Stufe:

(-)-(1S,2S)-3-(2-Dimethylaminomethyl-1-fluoro-cyclohexyl)-phenol, Hydrochlorid (**-1**)

7,75 g (22,7 mmol) (**-3**) wurden in 40 ml trockenem Methanol gelöst und in einer Hydrierapparatur mit 2,0 g Palladium auf Aktivkohle (10 % Pd) versetzt. Nach 35 Minuten Rühren bei Raumtemperatur waren 430 ml Wasserstoff verbraucht. Der Katalysator wurde durch Filtration und Methanol durch Destillation entfernt. Es wurden 6,3 g Base

gewonnen, aus der mit Trimethylchlorsilan/Wasser in 2-Butanon/Aceton (1/1) 4,9 g (75 % der Theorie) Hydrochlorid (-**1**) erhalten wurden.

Schmp: 188 -190° C
$[\alpha]_D^{RT}$ = -29,6° (c = 1,02; Methanol)

**Beispiel 2**

(+)-(1R,2R)-3-(2-Dimethylaminomethyl-1-fluoro-cyclohexyl)-phenol, Hydrochlorid (**+1**)

Ausgehend von (+)-(1R,2R)-3-(2-Dimethylaminomethyl-1-hydroxy-cyclohexyl)-phenol, Hydrochlorid wurde unter den in Beispiel 1 angegebenen Bedingungen das Enantiomere (**+1**) in einer Ausbeute von 48 % der Theorie erhalten.

Schmp: 188 -190° C
$[\alpha]_D^{RT}$ = +28,3° (c = 1,00; Methanol)

**Beispiel 3**

(+)-(1R,2R)-[2-Chloro-2-(3-methoxy-phenyl)-cyclohexylmethyl]-dimethylamin, Hydrochlorid (**+4**)

10 g (33,4 mmol) (+)-(1R,2R)-2-Dimethylaminomethyl-1-(3-methoxy-phenyl)-cyclohexanol, Hydrochlorid wurden bei Raumtemperatur mit 10 ml Thionylchlorid versetzt. Zur Entfernung von überschüssigem Thionylchlorid wurde anschließend zwei Stunden Stickstoff über das Reaktionsgemisch geleitet. Nach erneuter Zugabe von 10 ml Thionylchlorid wurde das Reaktionsgemisch 12 Stunden stehen gelassen, bevor innerhalb von 2,5 Stunden überschüssiges Thionylchlorid mit Hilfe eines Stickstoffstromes erneut entfernt wurde. Nach Trocknung wurde der Rückstand in 50 ml eiskaltem 2-Butanon gelöst und unter Rühren mit 50 ml Diisopropylether versetzt, wobei das Hydrochlorid auskristallisierte. Zur Vervollständigung wurde die Suspension noch zwei Stunden unter Eisbadkühlung gerührt. Es wurden 5,9 g (55 % der Theorie) (**+4**) erhalten.

Schmp: 120 -121° C (Zersetzung)
$[\alpha]_D^{RT}$ = +4,7° (c = 0,91; Methanol)

**Beispiel 4**

(-)-(1S,2S)-[2-Chloro-2-(3-methoxy-phenyl)-cyclohexylmethyl]-dimethylamin, Hydrochlorid (**-4**)

Ausgehend von (-)-(1S,2S)-2-Dimethylaminomethyl-1-(3-methoxy-phenyl)-cyclohexanol, Hydrochlorid wurde unter den in Beispiel 3 angegebenen Bedingungen das Enantiomere (**-4**) in einer Ausbeute von 55 % der Theorie erhalten.

Schmp: 120 -122° C
$[\alpha]_D^{RT}$ = -5,2° (c = 0,93; Methanol)

**Beispiel 5**

(+)-(1R,2R)-3-(1-Chloro-2-dimethylaminomethyl-cyclohexyl)-phenol, Hydrochlorid (**+5**)

3,6 g (12,6 mmol) (+)-(1S,2S)-3-(2-Dimethylaminomethyl-1-hydroxy-cyclohexyl)-phenol, Hydrochlorid wurden bei Raumtemperatur mit 3 ml Thionylchlorid versetzt. Anschließend wurde eine Stunde bei Raumtemperatur gerührt. Zur Entfernung von überschüssigem Thionylchlorid wurde dann zwei Stunden Stickstoff über das Reaktionsgemisch geleitet. Nach erneuter Zugabe von 4 ml Thionylchlorid wurde zwei Stunden bei Raumtemperatur gerührt, bevor innerhalb von zwei Stunden überschüssiges Thionylchlorid mit Hilfe eines Stickstoffstromes entfernt wurde. Der Rückstand wurde in 70 ml 2-Butanon gelöst und unter Rühren mit 50 ml Diisopropylether versetzt. Das auskristallisierte Hydrochlorid wurde dreimal mit 25 ml 2-Butanon dekantierend gewaschen. Nach Trocknung wurden 1,8 g (46 % der Theorie) (**+5**) erhalten.

Schmp: 145 -146°C (Zersetzung)

$[\alpha]_D^{RT}$ = +5,2° (c = 0,93; Methanol)

**Beispiel 6**

(-)-(1S,2S)-3-(1-Chloro-2-dimethylaminomethyl-cyclohexyl)-phenol, Hydrochlorid (**-5**)

Ausgehend von (-)-(1S,2S)-3-(2-Dimethylaminomethyl-1-hydroxy-cyclohexyl)-phenol, Hydrochlorid wurde unter den in Beispiel 5 angegebenen Bedingungen das Enantiomere (**-5**) in einer Ausbeute von 48 % der Theorie erhalten.

Schmp: 146 -147° C (Zersetzung)
$[\alpha]_D^{RT}$ = - 7,8° (c = 1,01; Methanol)

**Beispiel 7**

(+)-(1S,2R)-[2-(3-Methoxy-phenyl)-cyclohexylmethyl]-dimethylamin, Hydrochlorid (**+6**)

46 g getrocknetes Zinkchlorid wurden in 580 ml trockenem Ether gelöst und anschließend zu einer Aufschlämmung von 31 g Natriumborhydrid in 1800 ml Ether getropft. Nach 12-stündigem Rühren wurden von der erhaltenen Zinkborhydrid/Natriumchlorid-Suspension 500 ml abdekantiert und tropfenweise zu 10,2 g (32 mmol) (**+4**) in 200 ml trockenem Ether gegeben. Das Reaktionsgemisch wurde 48 Stunden bei Raumtemperatur gerührt und anschließend unter Eisbadkühlung tropfenweise mit 40 ml einer gesättigten Ammoniumchloridlösung versetzt. Nach Phasentrennung wurde die Etherphase zweimal mit gesättigter Kochsalzlösung gewaschen und nach dem Trocknen über Natriumsulfat das Lösungsmittel im Vakuum abdestilliert. Es wurden 9,6 g eines Amin-Boran-Komplexes erhalten, der zur Isolierung der freien Base in 100 ml trockenem Methanol gelöst wurde. Nach Zugabe von 7,5 g Triphenylphosphin wurde 18 Stunden unter Rückfluß erhitzt. Nach destillativer Entfernung des Lösungsmittels wurde der Rückstand mit 100 ml 5 %-iger Salzsäure versetzt, wobei anschließend die Salzsäurephase noch zweimal mit 50 ml Ether gewaschen wurde. Danach wurde die Salzsäurephase mit konzentrierter Natronlauge unter Eisbadkühlung alkalisiert und zweimal mit 50 ml Dichlormethan ausgeschüttelt. Nach Trocknung der vereinigten organischen Phasen über Natriumsulfat wurde das Lösungsmittel im Vakuum abdestilliert und der verbleibende Rückstand (7,8 g) in 2-Butanon aufgenommen. Nach der Zugabe von Trimethylchlorsilan/Wasser kristallisierten 6,9 g (76 % der Theorie) Hydrochlorid (**+6**) aus.

Schmp: 203 -204° C (Zersetzung)
$[\alpha]_D^{RT}$ = +68,0° (c = 1,00; Methanol)

**Beispiel 8**

(-)-(1R,2S)-[2-(3-Methoxy-phenyl)-cyclohexylmethyl]-dimethylamin, Hydrochlorid (**-6**)

Ausgehend von 10,2 g (32 mmol) (**-4**) wurde unter den in Beispiel 7 angegebenen Bedingungen das Enantiomere (**-6**) in einer Ausbeute von 75 % der Theorie erhalten.

Schmp: 201 -203° C (Zersetzung)
$[\alpha]_D^{RT}$ = - 67,1° (c = 1,0; Methanol)

**Beispiel 9**

(+)-(1S,2R)-3-(2-Dimethylaminomethyl-cyclohexyl)-phenol, Hydrochlorid (**+7**)

4,3 g (15 mmol) (**+6**), erhalten gemäß Beispiel 7, wurden mit 100 ml konzentrierter Bromwasserstoffsäure versetzt. Anschließend wurde zwei Stunden unter Rückfluß erhitzt. Nach dem Abkühlen auf Raumtemperatur wurde das Reaktionsgemisch im Wasserstrahlvakuum eingeengt. Der Rückstand wurde bis zur alkalischen Reaktion mit konzentrierter Natriumhydrogencarbonatlösung versetzt. Nach zweimaliger Extraktion mit je 50 ml Dichlormethan wurden die vereinigten organischen Phasen über Natriumsulfat getrocknet. Anschließend wurde Dichlormethan im Vakuum abdestilliert und der Rückstand (4 g) in 2-Butanon aufgenommen. Nach der Zugabe von Trimethylchlorsilan/Wasser kristallisierten 3,96 g (98 % der Theorie) Hydrochlorid (**+7**) aus.

Schmp: 177 -178° C (Zersetzung)

$[\alpha]_D^{RT}$ = +67,5° (c = 1,0; Wasser)

**Beispiel 10**

(-)-(1R,2S)-3-(2-Dimethylaminomethyl-cyclohexyl)-phenol, Hydrochlorid (**-7**)

Ausgehend von (**-6**), hergestellt nach Beispiel 8, wurde das Enantiomere (**-7**) unter den in Beispiel 9 angegebenen Bedingungen in 95 %iger Ausbeute erhalten.

Schmp: 174 -176° C (Zersetzung)
$[\alpha]_D^{RT}$ = -66,1° (c = 0,96; Methanol)

**Beispiel 11**

(-)-(1R,2S)-2,2-Dimethyl-propionsäure-3-(2-dimethylaminomethyl-cyclohexyl)-phenylester, Hydrochlorid (**-8**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung Dichlormethan destillativ entfernt. 1,7 g (7,3 mmol) der erhaltenen Base wurden in 10 ml trockenem Dimethylformamid gelöst und zu einer Suspension von 400 mg Natriumhydrid (50 %ig) in 5 ml trockenem Dimethylformamid getropft. Anschließend wurde noch 30 Minuten bei 50° C gerührt. Nach Abkühlen auf Raumtemperatur wurden 1,03 ml (8,4 mmol) 2,2-Dimethyl-propionylchlorid zugetropft und weitere zwei Stunden bei Raumtemperatur gerührt, bevor das Reaktionsgemisch auf Eis/Wasser gegossen wurde. Die wäßrige Phase wurde dreimal mit 50 ml Ether extrahiert. Die vereinigten organischen Phasen wurden über Natriumsulfat getrocknet. Nach destillativer Entfernung des Lösungsmittels wurden 2,3 g Rohgemisch gewonnen, das auf eine 4 x 30 cm Säule, gefüllt mit Kieselgel, gegeben wurde. Die Elution mit Diisopropylether/Methanol = 7/1 ergab 1,75 g Base, aus der mit Trimethylchlorsilan/Wasser in 2-Butanon/Diisopropylether 1,75 g (70 % der Theorie) Hydrochlorid (**-8**) mit einem Schmelzpunkt von 218 - 219° C erhalten wurden.

$[\alpha]_D^{RT}$ = -3,7° (c = 1,07; Methanol)

**Beispiel 12**

(-)-(1R,2S)-{2-[3-(p-Isopropyl-phenyl-carbamoyl)-oxy-phenyl]-cyclohexyl-methyl}-dimethylamin, Hydrochlorid (**-9**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung Dichlormethan destillativ entfernt. 2,1 g (9,0 mmol) der erhaltenen Base wurden in 20 ml trockenem Toluol gelöst und mit 1,62 g (10 mmol) 4-Isopropylphenylisocyanat versetzt. Nach 20stündigem Rühren bei Raumtemperatur wurde Toluol destillativ entfernt. Der Rückstand (2,5 g) wurde auf eine 5,5 x 15 cm Säule, gefüllt mit Kieselgel, gegeben und mit Methanol/Ethylacetat = 1/1 eluiert. Es wurden 1,94 g Base gewonnen, aus der mit Trimethylchlorsilan/Wasser in n-Propylacetat 1,8 g (46 % der Theorie) Hydrochlorid (**-9**) erhalten wurden.

Schmp: 156° C
$[\alpha]_D^{RT}$ = -16,3° (c = 1,09; Methanol)

**Beispiel 13**

(-)-(1R,2S)-2-Acetoxy-benzoesäure-3-(2-dimethylaminomethyl-cyclohexyl)-phenylester, Hydrochlorid (**-10**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung das Dichlormethan destillativ entfernt. 0,7 g (3,0 mmol) der erhaltenen Base wurden in 7 ml trockenem Dichlormethan gelöst und bei Raumtemperatur mit 0,6 g (3,24 mmol) 2-Acetyl-benzoyl-chlorid, gelöst in 3 ml trokkenem Dichlormethan, versetzt. Nach 20-stündigem Rühren bei Raumtemperatur wurde das Reaktionsgemisch mit 20 ml Natriumhydrogencarbonatlösung versetzt und die wäßrige Phase zweimal mit 10 ml Dichlormethan extrahiert. Die organischen Phasen wurden vereinigt und über Natriumsulfat getrocknet. Nach destillativer Entfernung des Lösungsmittels wurden 1,1 g Rohgemisch gewonnen, das auf eine 3 x 8 cm Säule, gefüllt mit Kieselgel, gegeben wurde. Die Elution mit Ether ergab 0,77 g Base, aus der mit Trimethylchlorsilan/Wasser in Ether 0,77 g (54 % der Theorie) Hydrochlorid (**-10**) erhalten wurden.

Schmp: 171 -174° C
$[\alpha]_D^{RT}$ = -27,6° (c = 1,15; Methanol)

**Beispiel 14**

(-)-(1R,2S)-Kohlensäure-[3-(2-dimethylaminomethyl-cyclohexyl)-phenyl]-ester-isobutylester, Hydrochlorid (**-11**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung Dichlormethan destillativ entfernt. 2,34 g (10 mmol) der erhaltenen Base wurden in 11 ml trockenem Dimethylformamid gelöst und zu einer Suspension von 0,54 g Natriumhydrid (50 %ig) in 5 ml trockenem Dimethylformamid getropft. Anschließend wurde 30 Minuten bei Raumtemperatur gerührt. Danach wurden 1,44 ml (11 mmol) Isobutyl-chloroformiat zugetropft und weitere zwei Stunden bei Raumtemperatur gerührt, bevor das Reaktionsgemisch mit 40 ml Wasser versetzt wurde. Die wäßrige Phase wurde dreimal mit 50 ml Ether extrahiert. Die vereinigten organischen Phasen wurden über Natriumsulfat getrocknet. Nach destillativer Entfernung des Lösungsmittels wurden 3,8 g Rohgemisch gewonnen, das auf eine 3 x 15 cm Säule, gefüllt mit Kieselgel, gegeben wurde. Die Elution mit Ether ergab 2,17 g Base, aus der mit Trimethylchlorsilan/Wasser in Ether 1,5 g (41 % der Theorie) Hydrochlorid (**-11**) als farbloser Sirup erhalten wurden.

$[\alpha]_D^{RT}$ = -33,7° (c = 1,16; Methanol)

**Beispiel 15**

(-)-(1R,2S)-4-Morpholin-4-yl-methyl-benzoesäure-3-(2-dimethylaminomethyl-cyclohexyl)-phenylester, Dihydrochlorid (**-12**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung Dichlormethan destillativ entfernt. 1,9 g (8,1 mmol) der erhaltenen Base wurden in 20 ml trockenem Dichlormethan gelöst und bei Raumtemperatur mit 2,2 g (9,2 mmol) 4-Morpho1in-4-yl-methyl-benzoylchlorid, Hydrochlorid (hergestellt nach US 4,623,486) versetzt. Nach 20-stündigem Rühren bei Raumtemperatur wurde das Reaktionsgemisch mit 50 ml Natriumhydrogencarbonatlösung versetzt und die wäßrige Phase dreimal mit 10 ml Dichlormethan extrahiert. Die organischen Phasen wurden vereinigt und über Natriumsulfat getrocknet. Nach destillativer Entfernung des Lösungsmittels wurden 2,9 g Rohgemisch gewonnen, das auf eine 4 x 20 cm Säule, gefüllt mit Kieselgel, gegeben wurde. Die Elution mit Diisopropylether/Methanol = 2/1 ergab 0,77 g Base, aus der mit Trimethylchlorsilan/Wasser in Ether 0,41 g (10 % der Theorie) Dihydrochlorid (**-12**) erhalten wurden.

Schmp: 234 -236° C
$[\alpha]_D^{RT}$ = - 26,8° (c = 1,00; Methanol)

**Beispiel 16:**

(+)-(2S,3S)-2-Amino-3-methyl-pentansäure-(1R,2S)-3-(2-dimethylaminomethyl-cyclohexyl)-phenyl-ester, Dihydrochlorid (**+13**)

1. Stufe:

(-)-(2S,3S)-2-tert.-Butoxycarbonylamino-3-methyl-pentansäure-(1R,2S)-3-(2-dimethylaminomethyl-cyclohexyl)-phenylester (**-14**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung das Dichlormethan destillativ entfernt. 2,1 g (9,8 mmol) der erhaltenen Base wurden in 140 ml trockenem Dichlormethan gelöst und bei Raumtemperatur nacheinander mit 2,19 g (9,5 mmol) (-)-(2S,3S)-2-tert-Butoxycarbonylamino-3-methyl-pentansäure, Monohydrat, 2,63 ml (19 mmol) Triethylamin und 4,94 g (9,5 mmol) Benzotriazol-1-yl-oxytripyrrolidino-phosphonium-hexafluorphosphat versetzt. Nach 2-stündigem Rühren bei Raumtemperatur wurde das Lösungsmittel abdestilliert und der Rückstand (10,1 g) auf eine 7 x 40 cm Säule, gefüllt mit Kieselgel, gegeben. Die Elution mit Ethylacetat/Methanol = 1/1 ergab 2,66 g Base (**-14**).

2. Stufe:

(+)-(2S,3S)-2-Amino-3-methyl-pentansäure-(1R,2S)-3-(2-dimethylaminomethyl-cyclohexyl)-phenyl-ester, Dihydrochlorid (**+13**)

2,66 g (5,7 mmol) (**-14**) wurden in 60 ml trockenem Dichlormethan gelöst und mit 0,23 ml Wasser (13 mmol) und 2,52 ml (19,5 mmol) Trimethylchlorsilan versetzt. Anschließend wurde 20 Stunden bei Raumtemperatur gerührt. Nach Zugabe von 100 ml Ether kristallisierten 2,1 g (56 % der Theorie) Hydrochlorid (+**13**) aus.

Schmp: 154° C (Zersetzung)
$[\alpha]_D^{RT}$ = +16,6°(c = 1,05; Methanol)

**Beispiel 17**

(-)-(1R,2S)-Dimethyl-{2-[3-(6-methyl-pyridin-2-yloxy)-phenyl]-cyclohexyl-methyl}-amin, Dihydrochlorid (**-15**)

Aus dem Enantiomeren (**-7**), hergestellt nach Beispiel 10, wurde mit Dichlormethan/wäßriger Natriumhydrogencarbonat-Lösung die Base freigesetzt und nach dem Trocknen der Lösung Dichlormethan destillativ entfernt. 2,1 g (9,0 mmol) der erhaltenen Base wurden in 10 ml trockenem Dimethylformamid gelöst und zu einer Suspension von 475 mg Natriumhydrid (50 %ig) in 5 ml trockenem Dimethylformamid getropft. Anschließend wurde 10 Minuten bei 60° C gerührt. Bei dieser Temperatur wurden 1,5 ml (13,7 mmol) 2-Chlor-6-methylpyridin zugetropft. Nach Zugabe von 30 mg Kupferpulver und 30 mg Kupfer-I-chlorid wurde 7 Stunden bei 140° C gerührt, bevor auf Raumtemperatur abgekühlt wurde. Das Reaktionsgemisch wurde mit 50 ml Wasser versetzt und die wäßrige Phase dreimal mit 50 ml Ether extrahiert. Die organischen Phasen wurden vereinigt, mit 10 ml Natronlauge und anschließend mit 10 ml Wasser gewaschen und über Natriumsulfat getrocknet. Nach destillativer Entfernung des Lösungsmittels wurden 3,2 g Rohgemisch gewonnen, das auf eine 5 x 20 cm Säule, gefüllt mit Kieselgel, gegeben wurde. Die Elution mit Ether/konzentrierter Ammoniaklösung = 99,5/0,5 ergab 1,0 g Base, aus der mit Trimethylchlorsilan/Wasser in 2-Butanon/Ethylacetat 1,89 g (53 % der Theorie) Dihydrochlorid (-**15**) erhalten wurden.

Schmp: 60° C (Sinterung)
$[\alpha]_D^{RT}$ = -44,6° (c = 1,0; Methanol)

**Beispiel 18**

(1RS,3SR,6RS)-6-Dimethylaminomethyl-1-(3-methoxy-phenyl)-cyclohexan-1,3-diol, Hydrochlorid (**16**)
und

(1RS,3RS,6RS)-6-Dimethylaminomethyl-1-(3-methoxy-phenyl)-cyclohexan-1,3-diol, Hydrochlorid (**17**)

1. Stufe:

9-Dimethylaminomethyl-3,3-dimethyl-1,5-dioxa-spiro[5.5]undecan-8-on, Hydrochlorid (**18**)

125 g (630 mmol) 3,3-Dimethyl-1,5-dioxa-spiro[5.5]undecan-8-on, welches durch azeotrope Acetalisierung von Cyclohexan-1,3-dion mit 2,2-Dimethyl-propan-1,3-diol in Toluol als Lösungsmittel unter Verwendung von p-Toluolsulfonsäure als Katalysator erhalten wurde, und 59 g (630 mmol) Dimethylammoniummethylenchlorid wurden in 400 ml trockenem Acetonitril bei Raumtemperatur gerührt. Nach Zugabe von 1 ml Acetylchlorid wurde 3 Stunden bei Raumtemperatur weitergerührt, wobei eine farblose, klare Lösung entstand. Anschließend wurden 800 ml trockener Ether zum Reaktionsgemisch getropft, wobei das Hydrochlorid auskristallisierte. Es wurden 158 g (98 % der Theorie) (**18**) erhalten.

2. Stufe

(8RS,9RS)-9-Dinethylaminomethyl-8-(3-methoxy-phenyl)-3.3-dimethyl-1,5-dioxo-spiro[5.5]undecan-8-ol (**19**)

Zu 3,88 g (160 mmol) Magnesiumspänen in 10 ml trockenem Tetrahydrofuran wurden tropfenweise 20 ml (158 mmol) 1-Brom-3-methoxy-benzol, gelöst in 100 ml trockenem Tetrahydrofuran, so zugegeben, daß das Reaktionsgemisch leicht siedete. Nach vollständiger Zugabe von 1-Brom-3-methoxybenzol wurde eine Stunde unter Rückfluß

erhitzt und danach auf 5 - 10° C abgekühlt. Aus dem Hydrochlorid (**18**) aus Stufe 1 wurde mit Dichlormethan/Natronlauge die Base freigesetzt und nach dem Trocknen der Lösung Dichlormethan destillativ entfernt. 32,7 g (150 mmol) der erhaltenen Base wurden in 50 ml trockenem Tetrahydrofuran gelöst und zur Grignard-Lösung gegeben. Das Reaktionsgemisch wurde über Nacht stehen gelassen und anschließend erneut auf 5 - 10° C abgekühlt. Durch Zugabe von 140 ml 20 %iger Ammoniumchloridlösung wurde die Grignardlösung zersetzt. Die Reaktionsmischung wurde mit 200 ml Ether/Tetrahydrofuran = 1/1 verdünnt, die organische Phase abgetrennt und zweimal mit 100 ml Ether extrahiert. Die vereinigten organischen Phasen wurden über Natriumsulfat getrocknet. Nach destillativer Entfernung des Lösungsmittels wurde der Rückstand (43,6 g) auf eine 8 x 50 cm Säule, gefüllt mit Kieselgel, gegeben und mit Ethylacetat/Methanol = 1/1 extrahiert. Die erhaltene Base wurde erneut auf eine 5 x 13 cm Säule, gefüllt mit Kieselgel, gegeben und mit Diisopropylether/Methanol = 1/1 eluiert. Es wurden 22,1 g (42 % der Theorie) Base als hellgelbes, viskoses Öl erhalten.

3. Stufe:

(3RS,4RS)-4-Dimethylaminomethyl-3-hydroxy-3-(3-methoxyphenyl)-cyclohexanon (**20**)

61,8 g (176 mmol) Base (**19**) aus Stufe 2 wurden in 800 ml Tetrahydrofuran gelöst und auf 0 - 5°C gekühlt. Bei dieser Temperatur wurden innerhalb von 30 Minuten 800 ml wäßrige Salzsäure (konz. Salzsäure/Wasser = 1/5) zugegeben. Anschließend wurde eine Stunde bei Raumtemperatur weitergerührt, bevor erneut auf 0 - 5° C abgekühlt wurde. Bei dieser Temperatur wurden 200 ml konzentrierte Natronlauge zugegeben. Das Reaktionsgemisch wurde dann dreimal mit 250 ml Ether extrahiert. Die vereinigten organischen Phasen wurden über Natriumsulfat getrocknet. Nach destillativer Entfernung des Lösungsmittels wurde der Rückstand (55 g) auf eine 8 x 50 cm Säule, gefüllt mit Kieselgel, gegeben und mit Diisopropylether/Methanol = 7/1 und später mit Ethylacetat/Methanol = 4/1 eluiert. Die erhaltene Base (24,7 g) wurde in 1.000 ml 2-Butanon aufgenommen und mit Trimethylchlorsilan/Wasser versetzt. Es kristallisierten 16,5 g (24 % der Theorie) Hydrochlorid (**20**) mit einem Schmelzpunkt von 161 - 163° C aus.

4. Stufe:

(1RS,3SR,6RS)-6-Dimethylaminomethyl-1-(3-methoxy-phenyl)-cyclohexan-1,3-diol, Hydrochlorid (**16**) und

(1RS,3RS,6RS)-6-Dimethylaminomethyl-1-(3-methoxy-phenyl)-cyclohexan-1,3-diol, Hydrochlorid (**17**)

Aus dem Hydrochlorid (**20**), hergestellt nach Stufe 3, wurde mit Dichlormethan/Natronlauge die Base freigesetzt und nach dem Trocknen der Lösung Dichlormethan destillativ entfernt. 27 g (97 mmol) der erhaltenen Base wurden in 300 ml Isopropanol gelöst und bei Raumtemperatur portionsweise mit 1,8 g (47,5 mmol) Natriumborhydrid versetzt. Es wurde eine Stunde bei Raumtemperatur gerührt, bevor auf 0 - 5° C abgekühlt wurde. Bei dieser Temperatur wurden 68 ml verdünnte Salzsäure (konz. Salzsäure/Wasser = 1/3) zugegeben. Unmittelbar nach der Zugabe wurde das Reaktionsgemisch mit konzentrierter Natronlauge alkalisiert. Nach destillativer Entfernung der Lösungsmittel wurde der Rückstand (40 g) in 200 ml Wasser aufgenommen und dreimal mit 50 ml Dichlormethan extrahiert. Die vereinigten organischen Phasen wurden über Natriumsulfat getrocknet und das Lösungsmittel destillativ entfernt. Der Rückstand (29,6 g) wurde auf eine 7 x 45 cm Säule, gefüllt mit Kieselgel, gegeben und zunächst mit Methanol und anschließend mit Methanol/konzentrierter Ammoniaklösung = 99,5/0,5 eluiert. Auf diese Weise konnten 11,3 g Base der Verbindung (**16**) und 13,5 g Base der Verbindung (**17**) erhalten werden. Die erhaltenen Basen wurden in 2-Butanon aufgenommen und mit Trimethylchlorsilan/Wasser versetzt, wobei die Hydrochloride auskristallisierten.

| | | |
|---|---|---|
| (**16**) | Ausbeute | 9,9 g (32 % der Theorie) |
| | Schmp. | 263 - 264° C |
| (**17**) | Ausbeute | 13,7 g (45 % der Theorie) |
| | Schmp. | 197 - 198° C |

**Beispiel 19**

Enantiomere von (**17**):

(+)-(1R,3R,6R)-6-Dimethylaminomethyl-1-(3-methoxy-phenyl)-cyclohexan-1,3-diol, Hydrochlorid (**+17**) und

(-)-(1S,3S,6S)-6-Dimethylaminomethyl-1-(3-methoxy-phenyl)-cyclohexan-1,3-diol, Hydrochlorid (**-17**)

Aus (**17**) wurde mit Dichlormethan/Natronlauge die Base freigesetzt. Nach dem Trocknen der Lösung wurde Dichlormethan im Vakuum abdestilliert. Das Racemat wurde dann auf der chiralen HPLC-Säule getrennt. Aus den erhaltenen Enantiomeren wurden durch Umsetzung mit Trimethylchlorsilan/Wasser in 2-Butanon die Hydrochloride mit einem Schmelzpunkt von 232 - 233° C hergestellt.

(**+17**): Ausbeute: 42 % der Theorie
$[\alpha]_D^{RT}$ = +14,0° (c = 1,12; Methanol)

(**-17**): Ausbeute: 44 % der Theorie
$[\alpha]_D^{RT}$ = -13,5° (c = 0,99; Methanol)

**Beispiel 20**

(1RS,3RS,6RS)-6-Dimethylaminomethyl-1-(3-hydroxy-phenyl)-cyclohexan-1,3-diol, Hydrochlorid (**21)**

Aus der nach Beispiel 18 erhaltenen Verbindung (**17**) wurde mit Dichlormethan/Natronlauge die Base freigesetzt und nach Trocknung der Lösung Dichlormethan destillativ entfernt. 8,06 g (28,8 mmol) Base wurden in 70 ml trockenem Toluol gelöst und langsam zu 120 ml (144 mmol) 1,2-molarer toluolischer Diisobutylaluminiumhydrid-Lösung getropft. Nach vollständiger Zugabe wurde 8 Stunden unter Rückfluß erhitzt und anschließend auf Raumtemperatur abgekühlt. Das Reaktionsgemisch wurde mit 50 ml Toluol verdünnt. Unter Eisbadkühlung wurden 13 ml Ethanol und anschließend 13 ml Wasser zugetropft. Nach einstündigem Rühren unter Eisbadkühlung wurde das Reaktionsgemisch durch Filterung von Aluminiumsalzen befreit, wobei der Rückstand dreimal mit je 50 ml Ethylacetat gewaschen wurde. Danach wurden die vereinigten organischen Phasen getrocknet und das Lösungsmittel destillativ entfernt. Aus der Base wurden in Aceton mit wäßriger Salzsäurelösung 7,3 g (84 % der Theorie) mit einem Schmelzpunkt von 226 - 228° C erhalten.

**Beispiel 21**

Enantiomere von (**21**):

(+)-(1R,3R,6R)-6-dimethylaminomethyl-1-(3-hydroxy-phenyl)-cyclohexan-1,3-diol, Hydrochlorid (+**21)** und

(-)-(1S,3S,6S)-6-dimethylaminomethyl-1-(3-hydroxy-phenyl)-cyclohexan-1,3-diol, Hydrochlorid (-**21**)

Aus (**21**) wurde mit Dichlormethan/wäßriger Natriumhydrogencarbonat-Lösung die Base freigesetzt. Nach dem Trocknen der Lösung wurde Dichlormethan im Vakuum abdestilliert. Das Racemat wurde dann auf der chiralen HPLC-Säule getrennt. Aus den erhaltenen Enantiomeren wurden in Aceton mit wäßriger Salzsäure die Hydrochloride mit einem Schmelzpunkt von 217 - 219°C hergestellt.

(**+21**): Ausbeute: 40 % der Theorie
$[\alpha]_D^{RT}$ +11,3° (c = 1,04; Methanol)

(**-21**): Ausbeute: 40 % der Theorie
$[\alpha]_D^{RT}$ 11,1° (c = 1,02; Methanol)

**Beispiel 22**

(1RS,2RS,5RS)-5-Benzyloxy-2-dimethylaminomethyl-1-(3-methoxy-phenyl)-cyclohexanol, Hydrochlorid (**22**)

Aus dem Hydrochlorid (**17**), hergestellt nach Beispiel 18, wurde mit Dichlormethan/Natronlauge die Base freigesetzt und nach dem Trocknen der Lösung Dichlormethan destillativ entfernt. 4,0 g (14,3 mmol) der erhaltenen Base wurden in 30 ml trockenem Dimethylformamid gelöst und zu einer Suspension von 690 mg Natriumhydrid (50 %ig) in 5 ml trockenem Dimethylformamid getropft. Anschließend wurde zwei Stunden bei Raumtemperatur gerührt. Nach Erwärmen auf 50° C wurden 1,81 g (14,3 mmol) Benzylchlorid zugetropft und weitere zwei Stunden bei 65° C sowie 15 Stunden bei Raumtemperatur gerührt. Anschließend wurde das Reaktionsgemisch auf Eis/Wasser gegossen. Die wäßrige Phase wurde dreimal mit 50 ml Ether extrahiert. Die organischen Phasen wurden vereinigt und über Natriumsulfat getrocknet. Nach destillativer Entfernung des Lösungsmittels wurden 4,6 g Rohgemisch gewonnen, das auf eine 4 x 30 cm Säule, gefüllt mit Kieselgel, gegeben wurde. Die Elution mit Ethylacetat/Methanol = 4/1 ergab 1,5 g Base, aus der mit Trimethylchlorsilan/Wasser in 2-Butanon/Diisopropylether 1,38 g (24 % der Theorie) Hydrochlorid (**22**) mit einem Schmelzpunkt von 138 - 139° C erhalten wurden.

**Beispiel 23**

(1RS,2RS,5SR)-2-Dimethylaminomethyl-1-(3-methoxy-phenyl)-5-methyl-cyclohexanol, Hydrochlorid (**23**)

95 ml (750 mmol) 1-Bromo-3-methoxy-benzol wurden in 425 ml trockenem Tetrahydrofuran gelöst und auf -75°C gekühlt. Nach Zugabe von 469 ml (750 mmol) 1,6 molarer n-Butyllithiumlösung in Hexan wurde eine Stunde bei -75° C gerührt. Anschließend wurden 82 g (484 mmol) (2RS,5SR)-Dimethylaminomethyl-5-methyl-cyclohexanon, hergestellt aus 3-Methylcyclohexanon, Dimethylaminhydrochlorid und Paraformaldehyd in Eisessig, gelöst in 120 ml trockenen Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 200 ml Wasser zugetropft, so daß die Innentemperatur 15° C nicht überstieg. Nach Phasentrennung wurde die wäßrige Phase dreimal mit 50 ml Ethylacetat extrahiert. Die vereinigten organischen Phasen wurden über Natriumsulfat getrocknet. Nach destillativer Entfernung des Lösungsmittels wurde der Rückstand (148,3 g) in 700 ml Aceton gelöst und mit Trimethylchlorsilan/Wasser versetzt. Bei 4 - 5°C kristallisierten 67 g (48 % der Theorie) Hydrochlorid (**23**) mit einem Schmelzpunkt von 173 - 175° C aus.

**Beispiel 24**

Enantiomere von (**23**):

(+)-(1R,2R,5S)-2-Dimethylaminomethyl-1-(3-methoxy-phenyl)-5-methyl-cyclohexanol, Hydrochlorid **(+23)**
und

(-)-(1S,2S,5R)-2-Dimethylaminomethyl-1-(3-methoxy-phenyl)-5-methyl-cyclohexanol, Hydrochlorid **(-23)**

Die Enantiomeren (**+23**) und (**-23**) wurden unter den in Beispiel 19 angegebenen Bedingungen hergestellt.

| | | |
|---|---|---|
| (**+23**) | Ausbeute: | 43 % der Theorie |
| | Schmp.: | 151 - 152° C |
| | $[\alpha]_D^{RT}$ = | +36,4° (c = 1,01; Methanol) |
| (**-23**) | Ausbeute: | 44 % der Theorie |
| | Schmp.: | 151 - 153° C |
| | $[\alpha]_D^{RT}$ = | -37,7° (c = 1,01 Methanol) |

**Beispiel 25**

(+)-(1R,2R,5S)-3-(2-Dimethylaminomethyl-1-hydroxy-5-methyl-cyclohexyl)-phenol, Hydrochlorid (**+24**)

Unter den in Beispiel 20 angegebenen Bedingungen wurde das Enantiomere (**+24**) aus der nach Beispiel 24 erhaltenen Methoxyverbindung (**+23**) hergestellt.

| | |
|---|---|
| Ausbeute: | 87 % der Theorie |
| Schmp.: | 221 - 223° C |
| $[\alpha]_D^{RT}$ = | +31,0° (c = 1,09; Methanol) |

**Beispiel 26**

(-)-(1S,2S,5R)-3-(2-Dimethylaminomethyl-1-hydroxy-5-methyl-cyclohexyl)-phenol, Hydrochlorid (**-24**)

Unter den in Beispiel 20 angegebenen Bedingungen wurde das Enantiomere (**-24**) aus der nach Beispiel 24 erhaltenen Methoxyverbindung (**-23**) hergestellt.

| | |
|---|---|
| Ausbeute: | 87 % der Theorie |
| Schmp.. | 220 - 222°C |
| $[\alpha]_D^{RT}$ = | +30,1° (c = 1,00; Methanol) |

**Beispiel 27**

(1RS,2RS,5SR)-3-(2-Dimethylaminomethyl-1-hydroxy-5-trifluoromethyl-cyclohexyl)-phenol, Hydrochlorid (**25**)

1. Stufe:

(1RS,2RS,5SR)-1-(3-Benzyloxy-phenyl)-2-dimethylaminomethyl-5-trifluoromethyl-cyclohexanol (**26**)

Zu 4,06 g (167 mmol) Magnesiumspänen in 40 ml trockenem Tetrahydrofuran wurden tropfenweise 43,9 g (167 mmol) 3-Benzyloxy-1-brombenzol, gelöst in 200 ml trockenem Tetrahydrofuran, so zugegeben, daß das Reaktionsgemisch leicht siedete. Nach vollständiger Zugabe von 3-Benzyloxy-1-brombenzol wurde eine Stunde unter Rückfluß erhitzt und danach auf 5 - 10° C abgekühlt. Bei dieser Temperatur wurden 30,8 g (139 mmol) (2RS,5SR)-2-Dimethylaminomethyl-5-trifluoromethyl-cyclohexanon, hergestellt aus 3-Trifluoromethyl-cyclohexanon und Dimethylaminomethylenchlorid in Acetonitril, gelöst in 80 ml trockenem Tetrahydrofuran, zugegeben. Das Reaktionsgemisch wurde über Nacht stehen gelassen und anschließend erneut auf 5 - 10° C abgekühlt. Durch Zugabe von 150 ml 20% iger Ammoniumchloridlösung wurde die Grignardlösung zersetzt. Die Reaktionsmischung wurde mit 200 ml Ether verdünnt, die organische Phase abgetrennt und die wäßrige Phase zweimal mit 100 ml Ether extrahiert. Die vereinigten organischen Phasen wurden über Natriumsulfat getrocknet. Nach destillativer Entfernung des Lösungsmittels wurde der Rückstand (60,6 g) auf eine 8 x 50 cm Säule, gefüllt mit Kieselgel, gegeben und mit Ethylacetat/Methanol eluiert. Es wurden 27,8 g (50 % der Theorie) Base (**26**) erhalten.

2. Stufe:

(1RS,2RS,5SR)-3-(2-Dimethylaminomethyl-1-hydroxy-5-trifluoromethyl-cyclohexyl)-phenol, Hydrochlorid (**25**)

Aus (**26**) aus Stufe 1 wurde (**25**) unter den in Beispiel 1 (Stufe 3) angegebenen Bedingungen in 64 %iger Ausbeute und einem Schmelzpunkt von 228 - 230° C erhalten.

**Beispiel 28:**

(1RS,2RS,5RS)-3-(2-Dimethylaminomethyl-1-hydroxy-5-trifluoromethyl-cyclohexyl)-phenol, Hydrochlorid (**27**)

Ausgehend von (2RS,5RS)-2-Dimethylaminomethyl-5-trifluoromethyl-cyclohexanon, hergestellt aus 3-Trifluoromethyl-cyclohexanon und Dimethylaminomethylenchlorid in Acetonitril, wurde unter den in Beispiel 27 angegebenen Bedingungen das zu Verbindung (**25**) 5-Epimere (**27**) in einer Ausbeute von 27 % der Theorie und einem Schmelzpunkt von 221 - 223°C erhalten.

**Beispiel 29**

(1RS,2RS,5SR)-3-(2-Dimethylaminomethy1-1-fluoro-5-trifluoromethyl-cyclohexyl)phenol, Hydrochlorid (**28**)

Ausgehend von der gemäß Beispiel 27 (Stufe 1) erhaltenen Base (**26**) wurde unter den in Beispiel 1, Stufen 2 und 3 angegebenen Bedingungen das Hydrochlorid (**28**) in einer Ausbeute von 24 % der Theorie und einem Schmelzpunkt von 204 - 205° C erhalten.

**Beispiel 30**

(1RS,2RS,5RS)-3-(2-Dimethylaminomethyl-1-fluoro-5-trifluoromethyl-cyclohexyl)-phenol, Hydrochlorid (**29**)

Ausgehend von der gemäß Beispiel 27 (Stufe 1) erhaltenen Base (1RS,2RS,5RS)-1-(3-Benzyloxy-phenyl)-2-dimethylaminomethyl-5-trifluoromethyl-cyclohexanol wurde unter den in Beispiel 29 angegebenen Bedingungen das Hydrochlorid (**29**) in einer Ausbeute von 22 % der Theorie und einem Schmelzpunkt von 204° C erhalten.

**Pharmakologische Untersuchungen**

**Analgesieprüfung im Tail-flick-Test an der Maus**

Die analgetische Wirksamkeit der erfindungsgemäßen Verbindungen wurde im Wärmestrahl (Tail-flick)-Test an der Maus nach der Methode von D'Amour und Smith (J. Pharm. Exp. Ther. 72, 74 - 79 (1941) untersucht. Dazu wurden NMRI-Mäuse mit einem Gewicht zwischen 20 und 24 g verwendet. Die Tiere wurden einzeln in spezielle Testkäfige gesetzt und die Schwanzbasis einem focussierten Wärmestrahl einer elektrischen Lampe (Rhema Analgesiemeter Typ 3010) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 - 5 Sekunden betrug. Vor Gabe einer erfindungsgemäßen Verbindung wurden die Tiere innerhalb von fünf Minuten zweimal vorgetestet und der Mittelwert dieser Messungen als Vortestmittelwert berechnet. Die Schmerzmessung wurde 20, 40 und 60 Minuten nach intravenöser Gabe durchgeführt. Bei Anstieg der Schmerzlatenz wurde die maximale Expositionszeit auf 12 Sekunden beschränkt und die Zunahme der Latenzzeit auf >150 % des Vortestmittelwerts als analgetische Wirkung gewertet. Zur Bestimmung der Dosisabhängigkeit wurde die jeweilige erfindungsgemäße Verbindung in 3 - 5 logarithmisch ansteigenden Dosen, die jeweils die Schwellen- und die maximale Wirkdosis einschlossen, appliziert und aus der Anzahl der analgetischen Tiere nach der Methode von Litchfield und Wilcoxon (J. Pharm. Exp. Ther. 96, 99 - 113 (1949)) die $ED_{50}$-Werte bestimmt. Die $ED_{50}$-Berechnung erfolgte im Wirkmaximum 20 Minuten nach intravenöser Substanzgabe.

Alle eingesetzten erfindungsgemäßen Verbindungen zeigten eine ausgeprägte analgetische Wirkung. Die Ergebnisse sind in der nachfolgenden Tabelle zusammengestellt.

Tabelle:

| Analgesieprüfung im Tail-flick-Test an der Maus | | |
|---|---|---|
| Beispiel | erfindungsgemäße Verbindung | $ED_{50}$ (mg/kg intravenös) |
| 1 | **(-1)** | 2,28 |
| 2 | **(+1)** | 0,64 |
| 5 | **(+5)** | 2,78 |
| 7 | **(+6)** | 10,70 |
| 9 | **(+7)** | 1,13 |
| 10 | **(-7)** | 5,90 |
| 11 | **(-8)** | 4,61 |
| 13 | **(-10)** | 8,71 |
| 14 | **(-11)** | 5,01 |
| 18 | **(17)** | 5,54 |
| 19 | **(+17)** | 3,93 |
| 21 | **(+21)** | 7,34 |
| Tramadol | - | 13,60 |

**Patentansprüche**

1. 6-Dimethylaminomethyl-1-phenyl-cyclohexanverbindungen der Formel I

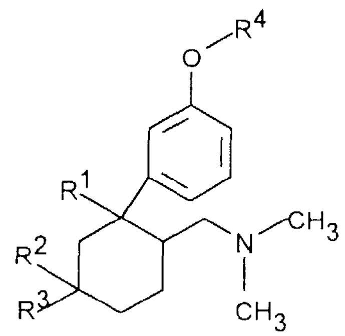

in der

$R^1$ H, OH, Cl oder F ist,

$R^2$ und $R^3$ gleich oder verschieden sind und H, $C_{1-4}$-Alkyl, Benzyl, $CF_3$, OH, $OCH_2$-$C_6H_5$, O-$C_{1-4}$-Alkyl, Cl oder F bedeuten mit der Maßgabe, daß wenigstens einer der Reste $R^2$ oder $R^3$ H bedeutet,

$R^4$ H, $CH_3$, PO(O$C_{1-4}$-Alkyl)$_2$, CO(O$C_{1-5}$-Alkyl), CO-NH-$C_6H_4$-$C_{1-3}$-Alkyl, CO-$C_6H_4$-$R^5$, CO-$C_{1-5}$-Alkyl, CO-CH$R^6$-NH$R^7$ oder eine unsubstituierte oder substituierte Pyridyl-, Thienyl-, Thiazoyl- oder Phenylgruppe bedeutet,

$R^5$ OC(O)$C_{1-3}$-Alkyl in ortho-Stellung oder $CH_2$-N($R^8$)$_2$ in meta- oder para-Stellung, wobei $R^8$ $C_{1-4}$-Alkyl oder beide Reste $R^8$ zusammen mit N den 4-Morpholino-Rest darstellen, bedeutet, und

$R^6$ und $R^7$ gleich oder verschieden sind und H oder $C_{1-6}$-Alkyl bedeuten,

mit der Maßgabe, daß wenn beide Reste $R^2$ und $R^3$ H bedeuten, $R^4$ nicht $CH_3$ ist, wenn $R^1$ H, OH oder Cl bedeutet oder $R^4$ nicht H ist, wenn $R^1$ OH bedeutet,

in Form ihrer Basen oder Salze von physiologisch verträglichen Säuren.

2. 6-Dimethylaminomethyl-l-phenyl-cyclohexanverbindungen gemäß Anspruch 1, dadurch gekennzeichnet, daß $R^1$ H, OH oder F ist.

3. 6-Dimethylaminomethyl-1-phenyl-cyclohexanverbindungen nach einem oder beiden der Ansprüche 1 bis 2, dadurch gekennzeichnet, daß die Verbindungen die Konfiguration der Formel Ia

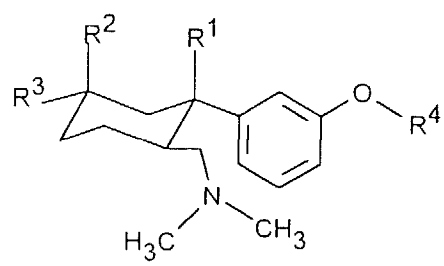

haben.

4. Verfahren zur Herstellung einer 6-Dimethylaminomethyl-1-phenyl-cyclohexanverbindung der Formel I

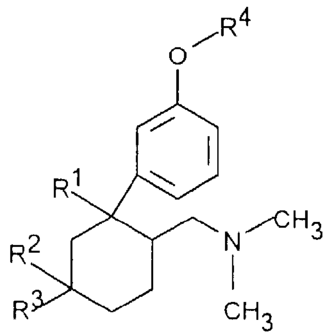

in der

$R^1$ OH ist,

$R^2$ und $R^3$ gleich oder verschieden sind und H, $C_{1-4}$-Alkyl, Benzyl, $CF_3$, Cl oder F bedeuten mit der Maßgabe, daß mindestens einer der Reste $R^2$ oder $R^3$ H bedeutet, und

$R^4$ H, $CH_3$, oder eine unsubstituierte oder substituierte Pyridyl-, Thienyl-, Thiazoyl- oder Phenylgruppe bedeutet,

mit der Maßgabe, daß $R^4$ weder $CH_3$ noch H ist, wenn beide Reste $R^2$ und $R^3$ H bedeuten,

dadurch gekennzeichnet, daß man ein β-Dimethylaminoketon der Formel II

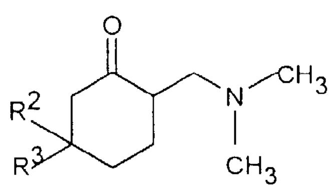

mit einer metallorganischen Verbindung der Formel III

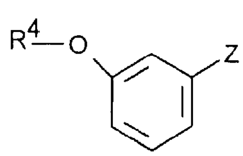

in der Z MgCl, MgBr, MgI oder Li bedeutet, zu einer Verbindung der Formel I, in der $R^1$ OH bedeutet, umsetzt.

**5.** Verfahren zur Herstellung einer 6-Dimethylaminomethyl-1-phenyl-cyclohexanverbindung der Formel I

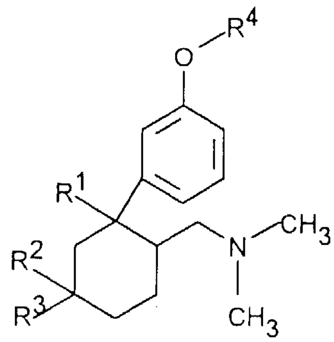

in der $R^1$ OH bedeutet,

einer der Reste $R^2$ oder $R^3$ H und der andere OH, O-$C_{1-4}$-Alkyl oder $OCH_2C_6H_5$ bedeutet, und

$R^4$ H, $CH_3$ oder eine unsubstituierte oder substituierte Pyridyl-, Thienyl-, Thiazoyl- oder Phenylgruppe bedeutet,

dadurch gekennzeichnet, daß man ein β-Dimethylaminoketon mit spirocyclischer Acetalstruktur der Formel V

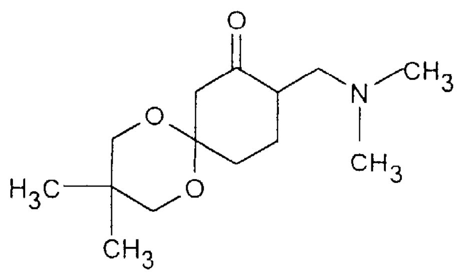

mit einer metallorganischen Verbindung der Formel III

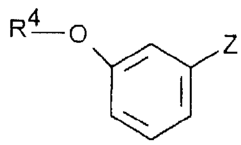

in der Z MgCl, MgBr, MgI oder Li bedeutet, zu einer Verbindung der Formel VI

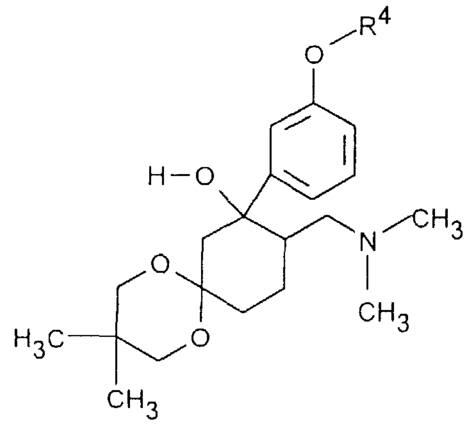

umsetzt, die erhaltene Verbindung durch protonenkatalysierte Deacetalisierung in ein Ketonderivat der Formel VIII

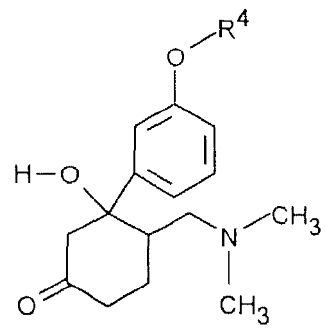

überführt und anschließend das erhaltene Ketonderivat mit einem komplexen Alkalimetallhydrid zu einer Verbindung der Formel I, in der einer der Reste $R^2$ oder $R^3$ OH bedeutet, reduziert und gewünschtenfalls die durch Reduktion erhaltene Verbindung der Formel I nach Überführung in ein Alkalisalz mit einem $C_{1-4}$-Alkyl- oder Benzylhalogenid zu einer Verbindung der Formel I, in der einer der Reste $R^2$ oder $R^3$ $OC_{1-4}$-Alkyl oder $OCH_2C_6H_5$ bedeutet, umsetzt.

**6.** Verfahren zur Herstellung einer 6-Dimethylaminomethyl-1-phenyl-cyclohexanverbindung der Formel I

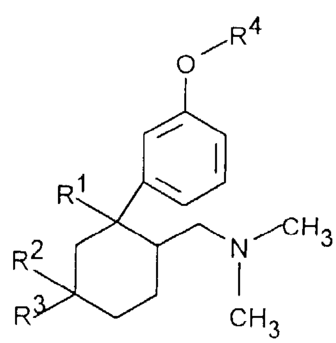

in der

$R^1$ H ist,

$R^2$ und $R^3$ gleich oder verschieden sind und H, $C_{1-4}$-Alkyl, Benzyl, $CF_3$, $OCH_2C_6H_5$ oder F bedeuten mit der Maßgabe, daß wenigstens einer der Reste $R^2$ oder $R^3$ H bedeutet, und

$R^4$ H, $CH_3$ oder eine unsubstituierte oder substituierte Pyridyl-, Thienyl-, Thiazoyl- oder Phenylgruppe bedeutet,

dadurch gekennzeichnet, daß man eine Verbindung der Formel I, in der $R^1$ Cl bedeutet, mit Zinkborhydrid, Zinkcyanoborhydrid oder Zinncyanoborhydrid in einem Ether oder eine Verbindung der Formel I, in der $R^1$ OH bedeutet, mit Raney-Nickel in einem $C_{2-4}$-Alkylalkohol umsetzt.

**7.** Verfahren zur Herstellung einer 6-Dimethylaminomethyl-1-phenyl-cyclohexanverbindung der Formel I

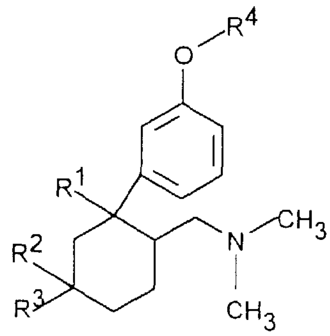

in der $R^1$ H ist,

$R^2$ und $R^3$ gleich oder verschieden sind und H, $C_{1-4}$-Alkyl, Benzyl, $CF_3$ oder F bedeuten mit der Maßgabe, daß wenigstens einer der Reste $R^2$ oder $R^3$ H ist, und

$R^4$ $CH_3$ bedeutet,

dadurch gekennzeichnet, daß man eine Verbindung der Formel I mit $R^1$ Cl in Gegenwart eines Palladiumkatalysators in einem $C_{1-4}$-Alkylalkohol hydriert.

**8.** Verfahren zur Herstellung einer 6-Dimethylaminomethyl-1-phenyl-cyclohexanverbindung der Formel I

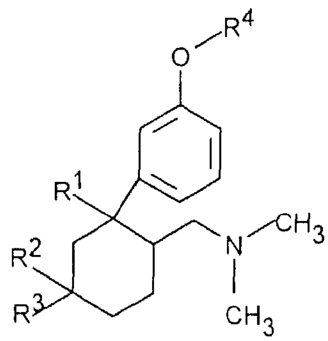

in der $R^1$ F ist,

$R^2$ und $R^3$ gleich oder verschieden sind und H, $C_{1-4}$-Alkyl, Benzyl, $CF_3$, $OCH_2$-$C_6H$, Cl oder F bedeuten mit der Maßgabe, daß wenigstens einer der Reste $R^2$ oder $R^3$ H ist, und

$R^4$ $CH_3$ oder eine unsubstituierte oder substituierte Pyridyl-, Thienyl-, Thiazoyl- oder Phenylgruppe bedeutet,

dadurch gekennzeichnet, daß man eine Verbindung der Formel I, in der $R^1$ OH ist, mit Dimethylaminoschwefeltrifluorid umsetzt.

**9.** Verwendung einer 6-Dimethylaminomethyl-1-phenyl-cyclohexanverbindung der Formel I gemäß Anspruch 1 als Wirkstoff zur Herstellung eines Arzneimittels.

**10.** Verwendung nach Anspruch 9, dadurch gekennzeichnet, daß das Arzneimittel ein Schmerzmittel ist.

## Claims

**1.** 6-dimethylaminomethyl-1-phenyl-cyclohexane compounds of formula I

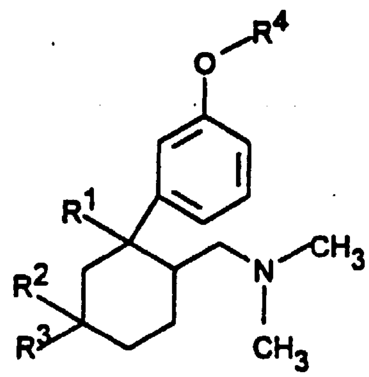

wherein

$R^1$ is H, OH, Cl or F,

$R^2$ and $R^3$ are the same or different and denote H, $C_{1-4}$-alkyl, benzyl, $CF_3$, OH, $OCH_2$-$C_6H_5$, O-$C_{1-4}$-alkyl, Cl or F, with the proviso that at least one of the $R^2$ or $R^3$ radicals denotes H,

$R^4$ denotes H, $CH_3$, PO($OC_{1-4}$-akyl)$_2$, CO($OC_{1-5}$-alkyl), CO-NH-$C_6H_4$-$C_{1-3}$-alkyl, CO-$C_6H_4$-$R^5$, CO-$C_{1-5}$-alkyl, CO-CH$R^6$-NH$R^7$ or an unsubstituted or substituted pyridyl, thienyl, thiazoyl or phenyl group,

$R^5$ denotes OC(O)$C_{1-3}$-alkyl in the ortho position or $CH_2$-N($R^8$)$_2$ in the meta or para position, wherein $R^8$ denotes $C_{1-4}$-alkyl, or both $R^8$ radicals, together with N, constitute a 4-morpholino radical, and

$R^6$ and $R^7$ are the same or different and denote H or $C_{1-6}$-alkyl,

with the proviso that, if both $R^2$ and $R^3$ radicals denote H, $R^4$ is not $CH_3$ if $R^1$ denotes H, OH or Cl, or $R^4$ is not H if $R^1$ denotes OH,

in the form of their bases or salts of physiologically compatible acids.

**2.** 6-dimethylaminomethyl-1-phenyl-cyclohexane compounds according to claim 1, characterised in that $R^1$ is H, OH or F.

**3.** 6-dimethylaminomethyl-1-phenyl-cyclohexane compounds according to one or both of claims 1 and 2, characterised in that the compounds have the configuration corresponding to formula Ia

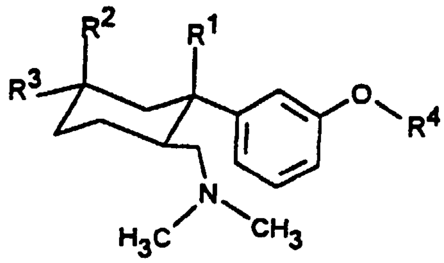

4. A method of producing a 6-dimethylaminomethyl-1-phenyl-cyclohexane compound of formula I

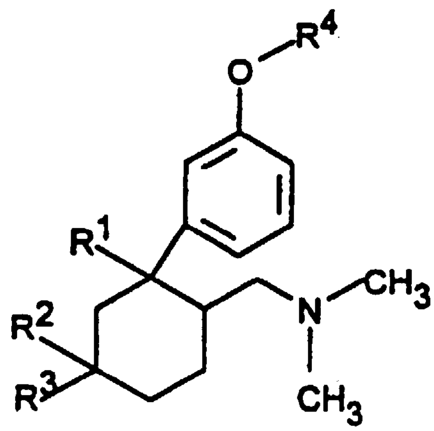

wherein

$R^1$ is OH,

$R^2$ and $R^3$ are the same or different and denote H, $C_{1-4}$-alkyl, benzyl, $CF_3$, Cl or F, with the proviso that at least one of the $R^2$ or $R^3$ radicals denotes H, and

$R^4$ denotes H, $CH_3$ or a unsubstituted or substituted pyridyl, thienyl, thiazoyl or phenyl group,

with the proviso that $R^4$ is neither $CH_3$ nor H if both $R^2$ and $R^3$ radicals denote H,

characterised in that a β-dimethylaminoketone of formula II

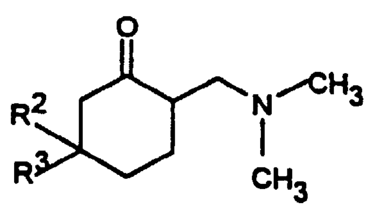

is reacted with an organometallic compound of formula III

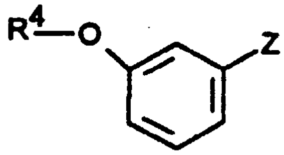

wherein Z denotes MgCl, MgBr, MgI or Li, to form a compound of formula I in which $R^1$ denotes OH.

**5.** A method of producing a 6-dimethylaminomethyl-1-phenyl-cyclohexane compound of formula I

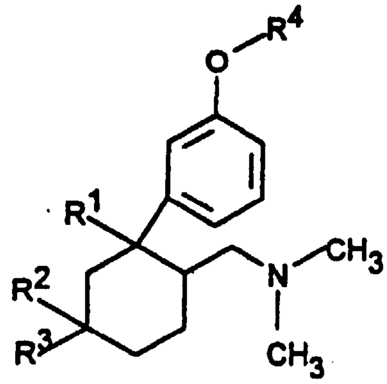

wherein $R^1$ denotes OH,

one of the $R^2$ or $R^3$ radicals denotes H and the other denotes OH, O-$C_{1-4}$-alkyl or $OCH_2C_6H_5$, and

$R^4$ denotes H, $CH_3$ or an unsubstituted or substituted pyridyl, thienyl, thiazoyl or phenyl group,

characterised in that a β-dimethylaminoketone with a spirocyclic acetal structure of formula V

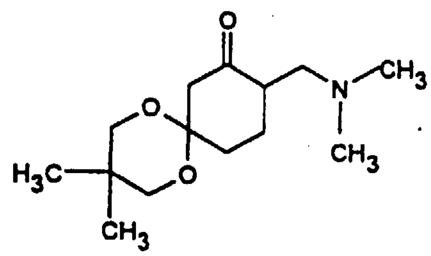

is reacted with an organometallic compound of formula III

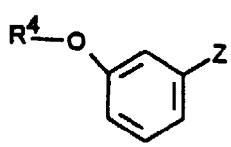

wherein Z denotes MgCl, MgBr, MgI or Li, to form a compound of formula VI

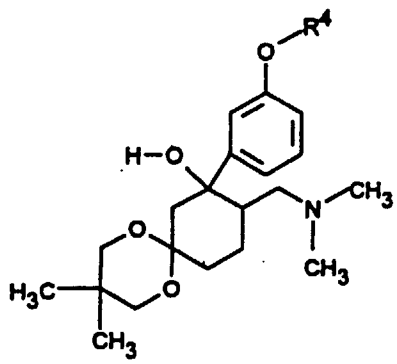

the compound obtained is converted by proton-catalysed deacetalisation into a ketone derivative of formula VIII

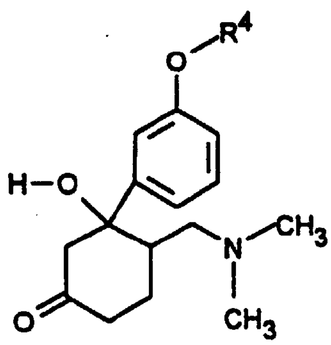

and the ketone derivative obtained is subsequently reduced by a complex alkali metal hydride to form a compound of formula I in which one of the $R^2$ or $R^3$ radicals denotes OH, and if desired the compound of formula I which is obtained by reduction is reacted, after conversion into an alkali salt with a $C_{1-4}$-alkyl or benzyl halide, to form a compound of formula I in which one of the $R^2$ or $R^3$ radicals denotes $OC_{1-4}$-alkyl or $OCH_2C_6H_5$.

6. A method of producing a 6-dimethylaminomethyl-1-phenyl-cyclohexane compound of formula I

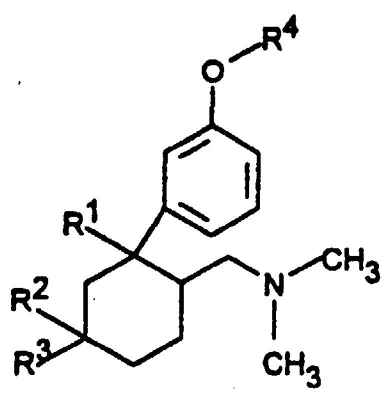

wherein

$R^1$ is H,

$R^2$ and $R^3$ are the same or different and denote H, $C_{1-4}$-alkyl, benzyl, $CF_3$, $OCH_2C_6H_5$ or F, with the proviso that at least one of the $R^2$ or $R^3$ radicals denotes H, and

$R^4$ denotes H, $CH_3$ or a unsubstituted or substituted pyridyl, thienyl, thiazoyl or phenyl group,

characterised in that a compound of formula I, in which $R^1$ denotes Cl, is reacted with zinc borohydride, zinc cyanoborohydride or tin cyanoborohydride in an ether, or a compound of formula I in which $R^1$ denotes OH is reacted with Raney nickel in a $C_{2-4}$-alkyl alcohol.

**7.** A method of producing a 6-dimethylaminomethyl-1-phenyl-cyclohexane compound of formula I

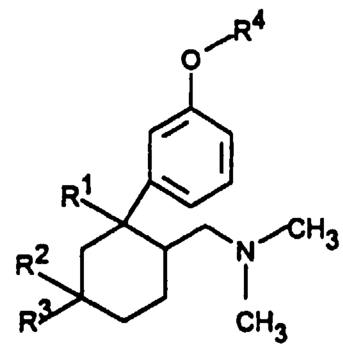

wherein $R^1$ is H,

$R^2$ and $R^3$ are the same or different and denote H, $C_{1-4}$-alkyl, benzyl, $CF_3$ or F, with the proviso that at least one of the $R^2$ or $R^3$ radicals is H, and

$R^4$ denotes $CH_3$,

characterised in that a compound of formula I, wherein $R^1$ is Cl, is hydrogenated in a $C_{1-4}$-alkyl alcohol in the presence of a palladium catalyst.

**8.** A method of producing a 6-dimethylaminomethyl-1-phenyl-cyclohexane compound of formula I

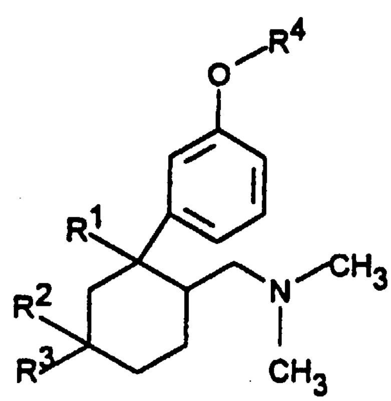

wherein $R^1$ is F,

$R^2$ and $R^3$ are the same or different and denote H, $C_{1-4}$-alkyl, benzyl, $CF_3$, $OCH_2$-$C_6H_5$, Cl or F, with the proviso that at least one of the $R^2$ or $R^3$ radicals is H, and

$R^4$ denotes $CH_3$ or an unsubstituted or substituted pyridyl, thienyl, thiazoyl or phenyl group,

characterised in that a compound of formula I, wherein $R^1$ is OH, is reacted with dimethylaminosulphur trifluoride.

**9.** The use of a 6-dimethylaminomethyl-1-phenyl-cyclohexane compound of formula I according to claim 1 as an

active ingredient for the production of a drug.

**10.** A use according to claim 9, characterised in that the drug is a pain-killing drug.

## Revendications

**1.** Composés de 6-diméthylaminométhyl-1-phénylcyclohexane de formule I

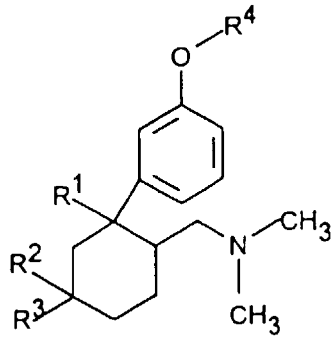

dans laquelle

$R^1$ représente H, OH, Cl ou F,
$R^2$ et $R^3$ sont identiques ou différents et représentent H, un groupe alkyle en $C_1$ à $C_4$, benzyle, $CF_3$, OH, $OCH_2$-$C_6H_5$, O- (alkyle en $C_1$ à $C_4$), Cl ou F, sous réserve que l'un au moins des restes $R^2$ ou $R^3$ représente H,
$R^4$ représente H, un groupe $CH_3$, PO-(O-alkyle en $C_1$ à $C_4$)$_2$, CO-(O-alkyle en $C_1$ à $C_5$), CO-NH-$C_6H_4$-(alkyle en $C_1$ à $C_3$), CO-$C_6H_4$-$R^5$, CO-(alkyle en $C_1$ à $C_5$), CO-CH$R^6$-NH$R^7$ ou un groupe pyridyle, thiényle, thiazoyle ou phényle non substitué ou substitué,
$R^5$ est un groupe OC(O)alkyle en $C_1$ à $C_3$ en position ortho ou un groupe $CH_2$-N($R^8$)$_2$ en position méta ou para, $R^8$ étant un groupe alkyle en $C_1$ à $C_4$ ou bien les deux restes $R^8$ représentant conjointement avec N le reste 4-morpholino, et
$R^6$ et $R^7$ sont identiques ou différents et représentent H ou un groupe alkyle en $C_1$ à $C_6$,
sous réserve que lorsque les deux restes $R^2$ et $R^3$ représentent H, $R^4$ ne soit pas un groupe $CH_3$ lorsque $R^1$ représente H, OH ou Cl ou que $R^4$ ne représente pas H lorsque $R^1$ est un groupe OH, sous forme de leurs bases ou de leurs sels d'acides acceptables du point de vue physiologique.

**2.** Composés de 6-diméthylaminométhyl-1-phénylcyclohexane suivant la revendication 1, caractérisés en ce que $R^1$ représente H, OH ou F.

**3.** Composés de 6-diméthylaminométhyl-1-phénylcyclohexane suivant l'une des revendications 1 et 2 ou les deux, caractérisés en ce que les composés ont la configuration de la formule Ia

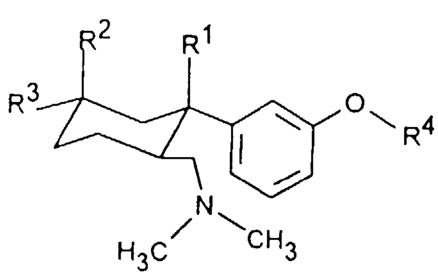

**4.** Procédé de production d'un composé de 6-diméthylaminométhyl-1-phényl-cyclohexane de formule I

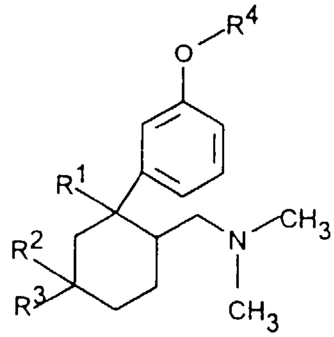

dans laquelle

$R^1$ représente OH,
$R^2$ et $R^3$ sont identiques ou différents et représentent H, un groupe alkyle en $C_1$ à $C_4$, benzyle, $CF_3$, Cl ou F, sous réserve que l'un au moins des restes $R^2$ ou $R^3$ représente H et
$R^4$ représente H, un groupe $CH_3$ ou un groupe pyridyle, thiényle, thiazoyle ou phényle non substitué ou substitué,
sous réserve que $R^4$ ne représente ni $CH_3$ ni H lorsque les deux restes $R^2$ et $R^3$ représentent H,

caractérisé en ce qu'on fait réagir une β-diméthylaminocétone de formule II

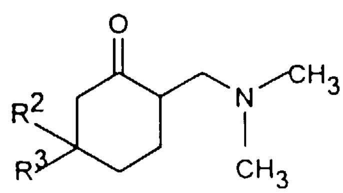

avec un composé organométallique de formule III

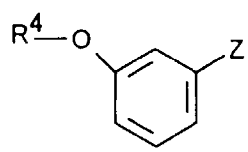

dans laquelle Z représente MgCl, MgBr, MgI ou Li, pour obtenir un composé de formule I dans laquelle $R^1$ représente OH.

**5.** Procédé de production d'un composé de 6-diméthylaminométhyl-1-phényl-cyclohexane de formule I

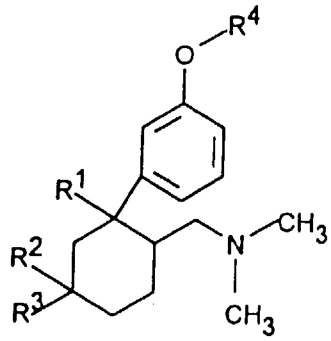

dans laquelle $R^1$ représente OH,

l'un des restes $R^2$ ou $R^3$ représente H et l'autre représente un groupe OH, O-(alkyle en $C_1$ à $C_4$) ou $OCH_2C_6H_5$ et
$R^4$ représente H, $CH_3$ ou un groupe pyridyle, thiényle, thiazoyle ou phényle non substitué ou substitué,

caractérisé en ce qu'on fait réagir une β-diméthylaminocétone à structure d'acétal spirocyclique de formule V

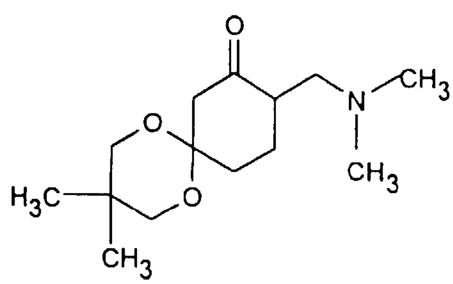

avec un composé organométallique de formule III

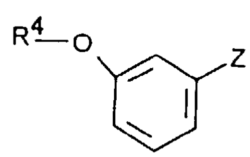

dans laquelle Z représente MgCl, MgBr, MgI ou Li, pour obtenir un composé de formule VI

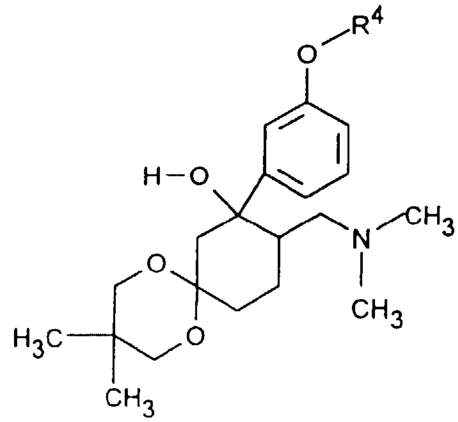

on transforme le composé obtenu par désacétalisation, catalysée par des protons, en un dérivé cétonique de formule VIII

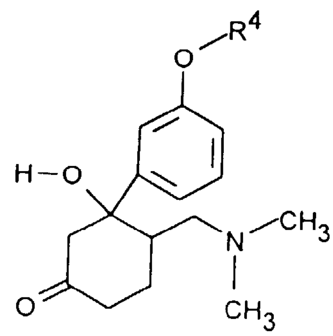

puis on réduit le dérivé cétonique obtenu avec un hydrure de métal alcalin complexe en un composé de formule I dans laquelle l'un des restes $R^2$ ou $R^3$ désigne OH, et on fait réagir le cas échéant le composé de formule I obtenu par réduction, après passage sous la forme d'un sel de métal alcalin, avec un halogénure d'alkyle en $C_1$ à $C_4$ ou de benzyle pour obtenir un composé de formule I dans laquelle l'un des restes $R^2$ ou $R^3$ désigne un groupe O-(alkyle en $C_1$ à $C_4$) ou $OCH_2C_6H_5$.

**6.** Procédé de production d'un composé de 6-diméthylaminométhyl-1-phényl-cyclohexane de formule I

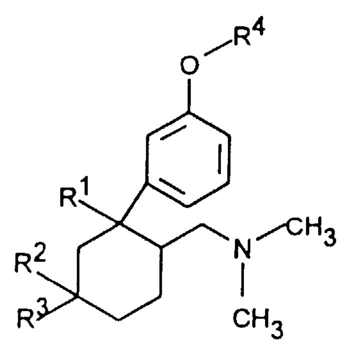

dans laquelle

$R^1$ représente H,
$R^2$ et $R^3$ sont identiques ou différents et représentent H, un groupe alkyle en $C_1$ à $C_4$, benzyle, $CF_3$, $OCH_2C_6H_5$ ou F, sous réserve que l'un au moins des restes $R^2$ ou $R^3$ représente H, et

$R^4$ représente H, un groupe $CH_3$ ou un groupe pyridyle, thiényle, thiazoyle ou phényle non substitué ou substitué,

caractérisé en ce qu'on fait réagir un composé de formule I, dans laquelle $R^1$ représente Cl, avec le borohydrure de zinc, le cyanoborohydrure de zinc ou le cyanoborohydrure d'étain dans un éther, ou un composé de formule I, dans laquelle $R^1$ est un groupe OH, avec le nickel de Raney dans un alcool alkylique en $C_2$ à $C_4$.

**7.** Procédé de production d'un composé de 6-diméthylaminométhyl-1-phényl-cyclohexane de formule I

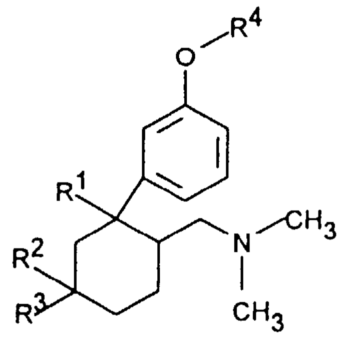

dans laquelle $R^1$ représente H,

$R^2$ et $R^3$ sont identiques ou différents et représentent H, un groupe alkyle en $C_1$ à $C_4$, benzyle, $CF_3$ ou F, sous réserve que l'un au moins des restes $R^2$ ou $R^3$ représente H, et
$R^4$ désigne un groupe $CH_3$,

caractérisé en ce qu'on hydrogène un composé de formule I dans laquelle $R^1$ représente Cl en présence d'un catalyseur au palladium dans un alcool alkylique en $C_1$ à $C_4$.

**8.** Procédé de production d'un composé de 6-diméthylaminométhyl-1-phényl-cyclohexane de formule I

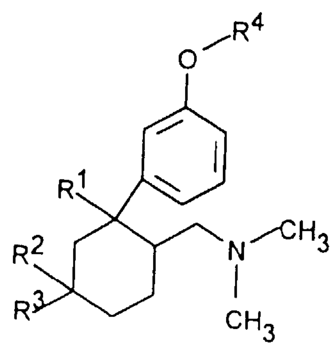

dans laquelle $R^1$ représente F,

$R^2$ et $R^3$ sont identiques ou différents et représentent H, un groupe alkyle en $C_1$ à $C_4$, benzyle, $CF_3$, $OCH_2$-$C_6H_5$, Cl ou F, sous réserve que l'un au moins des restes $R^2$ ou $R^3$ représente H, et
$R^4$ est un groupe $CH_3$ ou un groupe pyridyle, thiényle, thiazoyle ou phényle non substitué ou substitué,

caractérisé en ce qu'on fait réagir un composé de formule I, dans laquelle $R^1$ représente OH, avec le trifluorure de diméthylaminosoufre.

**9.** Utilisation d'un composé de 6-diméthylaminométhyl-1-phényl-cyclohexane de formule I suivant la revendication 1 comme substance active pour la préparation d'un médicament.

**10.** Utilisation suivant la revendication 9, caractérisée en ce que le médicament est un analgésique.